# EUROPEAN PATENT APPLICATION

(11) **EP 2 746 389 A1**
(43) Date of publication of application: **25.06.2014**
(21) Application number: 12824241.9
(22) Date of filing: 17.08.2012
(51) Int. Cl.: C12N 9/02, C12N 9/10, C12N 1/21, C12P 7/64, C12N 9/16

(54) **PRODUCTION OF BIODIESEL FROM GLYCERINE**

(30) Priority: 18.08.2011 ES
(71) Applicant: Idén Biotechnology, S.L., 31002 Pamplona (Navarra) (ES)
(72) Inventor: SCHUJMAN, Gustavo Enrique, 31002 Pamplona (ES); DE MENDOZA, Diego, 31002 Pamplona (ES)
(74) Representative: Illescas, Manuel
(86) International application number: PCT/ES2012/070628
(87) International publication number: WO 2013/024196

(57) **Abstract**

The present invention describes bacterial strains CECT 7968, CECT 7969 and NCIMB 42026 of the species *B. subtilis,* capable of expressing the heterologous synthetic mutated genes: *pdc* and *adhB,* originating from Z. *mobilis, 'tesA,* originating from *E. coli,* and *atf1,* originating from *Acinetobacter sp.* ADP1. Furthermore, said strains may overexpress at least one of the genes of the ACC (acetyl-CoA carboxylase) and acyl-CoA synthetase enzymatic complexes. The use of said strains produces an increase in the production of biofuel, preferably biodiesel from glycerin as the carbon source. Moreover, the present invention describes the use of said bacterial strains for the production of said biofuel, biodiesel, from glycerin, as well as a process for synthesising biofuel, preferably biodiesel, using the strains described in the present invention and the biofuel duly obtained.

SEQUENCE LISTING

<110>  Iden Biotechnology S.L.

<120>  Production of biodiesel from glycerine

<130>  PCT-05681

<160>  22

<170>  PatentIn version 3.5

<210>  1
<211>  1707
<212>  DNA
<213>  Zymomonas mobilis

<400>  1
atgagttata ctgtcggtac ctatttagcg gagcggcttg tccagattgg tctcaagcat      60

cacttcgcag tcgcgggcga ctacaacctc gtccttcttg acaacctgct tttgaacaaa     120

aacatggagc aggtttattg ctgtaacgaa ctgaactgcg gtttcagtgc agaaggttat     180

gctcgtgcca aaggcgcagc agcagccgtc gttacctaca gcgtcggtgc gctttccgca     240

tttgatgcta tcggtggcgc ctatgcagaa aaccttccgg ttatcctgat ctccggtgct     300

ccgaacaaca atgaccacgc tgctggtcac gtgttgcatc acgctcttgg caaaaccgac     360

tatcactatc agttggaaat ggccaagaac atcacggccg ccgctgaagc gatttatacc     420

ccggaagaag ctccggctaa aatcgatcac gtgattaaaa ctgctcttcg tgagaagaag     480

ccggtttatc tcgaaatcgc ttgcaacatt gcttccatgc cctgcgccgc tcctggaccg     540

gcaagcgcat tgttcaatga cgaagccagc gacgaagctt ctttgaatgc agcggttgaa     600

gaaaccctga aattcatcgc caaccgcgac aaagttgccg tcctcgtcgg cagcaagctg     660

cgcgcagctg gtgctgaaga agctgctgtc aaatttgctg atgctcttgg tggcgcagtt     720

gctaccatgg ctgctgcaaa aagcttcttc ccagaagaaa acccgcatta catcggtacc     780

tcatggggtg aagtcagcta tccgggcgtt gaaaagacga tgaaagaagc cgatgcggtt     840

atcgctctgg ctcctgtctt taacgactac tccaccactg gttggacgga tattcctgat     900

cctaagaaac tggttctcgc tgaaccgcgt tctgtcgtcg ttaacggcat tcgcttcccc     960

agcgtccatc tgaaagacta tctgacccgt ttggctcaga aagtttccaa gaaaaccggt    1020

gctttggact tcttcaaatc cctcaatgca ggtgaactga agaaagccgc tccggctgat    1080

ccgagtgctc cgttggtcaa cgcagaaatc gcccgtcagg tcgaagctct tctgaccccg    1140

aacacgacgg ttattgctga aaccggtgac tcttggttca atgctcagcg catgaagctc    1200

ccgaacggtg ctcgcgttga atatgaaatg cagtggggtc acattggttg gtccgttcct    1260

gccgccttcg gttatgccgt cggtgctccg gaacgtcgca acatcctcat ggttggtgat    1320

ggttccttcc agctgacggc tcaggaagtc gctcagatgg ttcgcctgaa actgccggtt    1380

atcatcttct tgatcaataa ctatggttac accatcgaag ttatgatcca tgatggtccg 1440

tacaacaaca tcaagaactg ggattatgcc ggtctgatgg aagtgttcaa cggtaacggt 1500

ggttatgaca gcggtgctgg taaaggcctg aaggctaaaa ccggtggcga actggcagaa 1560

gctatcaagg ttgctctggc aaacaccgac ggcccaaccc tgatcgaatg cttcatcggt 1620

cgtgaagact gcactgaaga attggtcaaa tggggtaagc gcgttgctgc cgccaacagc 1680

cgtaagcctg ttaacaagct cctctag 1707

<210> 2
<211> 1707
<212> DNA
<213> Artificial Sequence

<220>
<223> synthetic sequence of gene pdc

<400> 2
atgtcttata ctgttggtac ttatttagct gaacgtcttg ttcaaattgg tcttaaacat 60

cacttcgcag ttgctggtga ctacaacctt gttcttcttg acaaccttct tcttaacaaa 120

aacatggaac aagtttattg ctgtaacgaa cttaactgcg gtttctctgc agaaggttat 180

gctcgtgcta aaggtgcagc agcagctgtt gttacttact ctgttggtgc tctttctgca 240

ttcgatgcta tcggtggtgc ttatgcagaa aaccttcctg ttatccttat ctctggtgct 300

cctaacaaca acgaccacgc tgctggtcac gttcttcatc acgctcttgg taaaactgac 360

tatcactatc aacttgaaat ggctaaaaac atcactgctg ctgctgaagc tatttatact 420

cctgaagaag ctcctgctaa aatcgatcac gttattaaaa ctgctcttcg tgaaaaaaaa 480

cctgtttatc ttgaaatcgc ttgcaacatt gcttctatgc cttgcgctgc tcctggacct 540

gcatctgcac ttttcaacga cgaagcatct gacgaagcat ctcttaacgc agctgttgaa 600

gaaactctta aattcatcgc taaccgcgac aaagttgctg ttcttgttgg ttctaaactt 660

cgcgcagctg gtgctgaaga agctgctgtt aaattcgctg atgctcttgg tggtgcagtt 720

gctactatgg ctgctgcaaa atctttcttc ccagaagaaa accctcatta catcggtact 780

tcttggggtg aagtttctta tcctggtgtt gaaaaaacta tgaaagaagc tgatgctgtt 840

atcgctcttg ctcctgtttt caacgactac tctactactg gttggactga tattcctgat 900

cctaaaaaac ttgttcttgc tgaacctcgt tctgttgttg ttaacggtat tcgcttccct 960

tctgttcatc ttaaagacta tcttactcgt cttgctcaaa aagtttctaa aaaaactggt 1020

gctcttgact tcttcaaatc tcttaacgca ggtgaactta aaaaagctgc tcctgctgat 1080

ccttctgctc ctcttgttaa cgcagaaatc gctcgtcaag ttgaagctct tcttactcct 1140

aacactactg ttattgctga aactggtgac tcttggttca acgctcaacg catgaaactt 1200

cctaacggtg ctcgcgttga atatgaaatg caatggggtc acattggttg gtctgttcct 1260

gctgctttcg gttatgctgt tggtgctcct gaacgtcgca acatccttat ggttggtgat 1320

ggttctttcc aacttactgc tcaagaagtt gctcaaatgg ttcgccttaa acttcctgtt 1380

atcatcttcc ttatcaacaa ctatggttac actatcgaag ttatgatcca tgatggtcct 1440

tacaacaaca tcaaaaactg ggattatgct ggtcttatgg aagttttcaa cggtaacggt 1500

ggttatgact ctggtgctgg taaaggtctt aaagctaaaa ctggtggtga acttgcagaa 1560

gctatcaaag ttgctcttgc aaacactgac ggtccaactc ttatcgaatg cttcatcggt 1620

cgtgaagact gcactgaaga acttgttaaa tggggtaaac gcgttgctgc tgctaactct 1680

cgtaaacctg ttaacaaact tctttag 1707

<210> 3
<211> 1152
<212> DNA
<213> Zymomonas mobilis

<400> 3
atggcttctt caacttttta tattcctttc gtcaacgaaa tgggcgaagg ttcgcttgaa 60

aaagcaatca aggatcttaa cggcagcggc tttaaaaatg cgctgatcgt ttctgatgct 120

ttcatgaaca aatccggtgt tgtgaagcag gttgctgacc tgttgaaagc acagggtatt 180

aattctgctg tttatgatgg cgttatgccg aacccgactg ttaccgcagt tctggaaggc 240

cttaagatcc tgaaggataa caattcagac ttcgtcatct ccctcggtgg tggttctccc 300

catgactgcg ccaaagccat cgctctggtc gcaaccaatg gtggtgaagt caaagactac 360

gaaggtatcg acaaatctaa gaaacctgcc ctgcctttga tgtcaatcaa cacgacggct 420

ggtacggctt ctgaaatgac gcgtttctgc atcatcactg atgaagtccg tcacgttaag 480

atggccattg ttgaccgtca cgttaccccg atggtttccg tcaacgatcc tctgttgatg 540

gttggtatgc caaaaggcct gaccgccgcc accggtatgg atgctctgac ccacgcattt 600

gaagcttatt cttcaacggc agctactccg atcaccgatg cttgcgcctt gaaggctgcg 660

tccatgatcg ctaagaatct gaagaccgct tgcgacaacg gtaaggatat gccagctcgt 720

gaagctatgg cttatgccca attcctcgct ggtatggcct tcaacaacgc ttcgcttggt 780

tatgtccatg ctatggctca ccagttgggc ggctactaca acctgccgca tggtgtctgc 840

aacgctgttc tgcttccgca tgttctggct tataacgcct ctgtcgttgc tggtcgtctg 900

aaagacgttg gtgttgctat gggtctcgat atcgccaatc tcggtgataa agaaggcgca 960

gaagccacca ttcaggctgt tcgcgatctg gctgcttcca ttggtattcc agcaaatctg 1020

accgagctgg gtgctaagaa agaagatgtg ccgcttcttg ctgaccacgc tctgaaagat 1080

gcttgtgctc tgaccaaccc gcgtcagggt gatcagaaag aagttgaaga actcttcctg 1140

agcgctttct aa                                                          1152

<210>  4
<211>  1152
<212>  DNA
<213>  Artificial Sequence

<220>
<223>  Synthetic sequence of gene adhB

<400>  4
atggcttctt ctactttcta tattcctttc gttaacgaaa tgggtgaagg ttctcttgaa      60

aaagcaatca aagaccttaa cggttctggt ttcaaaaacg ctcttatcgt ttctgatgct     120

ttcatgaaca aatctggtgt tgttaaacaa gttgctgacc ttcttaaagc acaaggtatt     180

aactctgctg tttatgatgg tgttatgcct aaccctactg ttactgcagt tcttgaaggt     240

cttaaaatcc ttaaagataa caactctgac ttcgttatct ctcttggtgg tggttctcct     300

catgactgcg ctaaagctat cgctcttgtt gcaactaacg gtggtgaagt taaagactac     360

gaaggtatcg acaaatctaa aaaacctgct cttcctctta tgtctatcaa cactactgct     420

ggtactgctt ctgaaatgac tcgtttctgc atcatcactg atgaagttcg tcacgttaaa     480

atggctattg ttgaccgtca cgttactcct atggtttctg ttaacgatcc tcttcttatg     540

gttggtatgc caaaaggtct tactgctgct actggtatgg atgctcttac tcacgcattc     600

gaagcatatt cttctactgc agctactcct atcactgatg cttgcgctct taaagctgct     660

tctatgatcg ctaaaaacct taaaactgct tgcgacaacg gtaaagatat gccagctcgt     720

gaagctatgg cttatgctca attccttgct ggtatggctt tcaacaacgc ttctcttggt     780

tatgttcatg ctatggctca ccaacttggt ggttactaca accttcctca tggtgtttgc     840

aacgctgttc ttcttcctca tgttcttgct tataacgctt ctgttgttgc tggtcgtctt     900

aaagacgttg gtgttgctat gggtcttgat atcgctaacc ttggtgataa agaaggtgca     960

gaagctacta ttcaagctgt tcgcgatctt gctgcttcta ttggtattcc agcaaacctt    1020

actgaacttg gtgctaaaaa agaagatgtt cctcttcttg ctgaccacgc tcttaaagat    1080

gcttgtgctc ttactaaccc tcgtcaaggt gatcaaaaag aagttgaaga acttttcctt    1140

tctgctttct aa                                                        1152

<210>  5
<211>  555
<212>  DNA
<213>  Escherichia coli

<400>  5
atggcagcgg acacgttatt gattctgggt gatagcctga gcgccgggta tcgaatgtct      60

gccagcgcgg cctggcctgc cttgttgaat gataagtggc agagtaaaac gtcggtagtt     120

aatgccagca tcagcggcga cacctcgcaa caaggactgg cgcgccttcc ggctctgctg 180

aaacagcatc agccgcgttg ggtgctggtt gaactgggcg gcaatgacgg tttgcgtggt 240

tttcagccac agcaaaccga gcaaacgctg cgccagattt tgcaggatgt caaagccgcc 300

aacgctgaac cattgttaat gcaaatacgt ctgcctgcaa actatggtcg ccgttataat 360

gaagccttta gcgccattta ccccaaactc gccaaagagt ttgatgttcc gctgctgccc 420

ttttttatgg aagaggtcta cctcaagcca caatggatgc aggatgacgg tattcatccc 480

aaccgcgacg cccagccgtt tattgccgac tggatggcga agcagttgca gcctttagta 540

aatcatgact cataa 555

<210> 6
<211> 555
<212> DNA
<213> Artificial Sequence

<220>
<223> Synthetic sequence of gene ´tesA

<400> 6
atggcagctg acactttact tattcttggt gattctcttt ctgctggtta tcgtatgtct 60

gcttctgctg cttggcctgc tcttcttaac gataaatggc aatctaaaac ttctgtagtt 120

aacgcttcta tctctggtga cacttctcaa caaggacttg ctcgccttcc tgctcttctt 180

aaacaacatc aacctcgttg ggttcttgtt gaacttggtg gtaacgacgg tcttcgtggt 240

ttccaaccac aacaaactga acaaactctt cgccaaattc ttcaagatgt taaagctgct 300

aacgctgaac cacttttaat gcaaatccgt cttcctgcaa actatggtcg ccgttataac 360

gaagcattct ctgctattta ccctaaactt gctaaagaat tcgatgttcc tcttcttcct 420

ttcttcatgg aagaagttta ccttaaacca caatggatgc aagatgacgg tattcatcct 480

aaccgcgacg ctcaaccttt cattgctgac tggatggcta aacaacttca acctttagta 540

aaccatgact cttaa 555

<210> 7
<211> 1377
<212> DNA
<213> Acinetobacter strain baylyi ADP1

<400> 7
atgcgcccat tacatccgat tgattttata ttcctgtcac tagaaaaaag acaacagcct 60

atgcatgtag gtggtttatt tttgtttcag attcctgata acgccccaga cacctttatt 120

caagatctgg tgaatgatat ccggatatca aaatcaatcc ctgttccacc attcaacaat 180

aaactgaatg ggcttttttg ggatgaagat gaagagtttg atttagatca tcattttcgt 240

catattgcac tgcctcatcc tggtcgtatt cgtgaattgc ttatttatat ttcacaagag 300

cacagtacgc tgctagatcg ggcaaagccc ttgtggacct gcaatattat tgaaggaatt 360

gaaggcaatc gttttgccat gtacttcaaa attcaccatg cgatggtcga tggcgttgct 420

ggtatgcggt taattgaaaa atcactctcc catgatgtaa cagaaaaaag tatcgtgcca 480

ccttggtgtg ttgagggaaa acgtgcaaag cgcttaagag aacctaaaac aggtaaaatt 540

aagaaaatca tgtctggtat taagagtcag cttcaggcga cacccacagt cattcaagag 600

ctttctcaga cagtatttaa agatattgga cgtaatcctg atcatgtttc aagctttcag 660

gcgccttgtt ctattttgaa tcagcgtgtg agctcatcgc gacgttttgc agcacagtct 720

tttgacctag atcgttttcg taatattgcc aaatcgttga atgtgaccat taatgatgtt 780

gtactagcgg tatgttctgg tgcattacgt gcgtatttga tgagtcataa tagtttgcct 840

tcaaaaccat taattgccat ggttccagcc tctattcgca atgacgattc agatgtcagc 900

aaccgtatta cgatgattct ggcaaatttg gcaacccaca aagatgatcc tttacaacgt 960

cttgaaatta tccgccgtag tgttcaaaac tcaaagcaac gcttcaaacg tatgaccagc 1020

gatcagattc taaattatag tgctgtcgta tatggccctg caggactcaa cataatttct 1080

ggcatgatgc caaaacgcca agccttcaat ctggttattt ccaatgtgcc tggcccaaga 1140

gagccacttt actggaatgg tgccaaactt gatgcactct acccagcttc aattgtatta 1200

gacggtcaag cattgaatat tacaatgacc agttatttag ataaacttga agttggtttg 1260

attgcatgcc gtaatgcatt gccaagaatg cagaatttac tgacacattt agaagaagaa 1320

attcaactat ttgaaggcgt aattgcaaag caggaagata ttaaaacagc caattaa 1377

<210> 8
<211> 1377
<212> DNA
<213> Artificial Sequence

<220>
<223> Synthetic sequence of gene atf1

<400> 8
atgcgcccat tacatcctat tgatttcatc ttcctttctc ttgaaaaacg tcaacaacct 60

atgcatgtag gtggtttatt ccttttccaa attcctgata acgctccaga cactttcatt 120

caagaccttg ttaacgatat ccgtatctct aaatctatcc ctgttccacc attcaacaac 180

aaacttaacg gtcttttctg ggatgaagat gaagaattcg atttagatca tcatttccgt 240

catattgcac ttcctcatcc tggtcgtatt cgtgaacttc ttatttatat ttctcaagaa 300

cactctactc ttcttgatcg tgcaaaacct ctttggactt gcaacattat tgaaggaatt 360

gaaggtaacc gtttcgctat gtacttcaaa attcaccatg ctatggttga tggtgttgct 420

ggtatgcgtt taattgaaaa atctctttct catgatgtaa cagaaaaatc tatcgttcca 480

ccttggtgtg ttgaaggaaa acgtgcaaaa cgcttacgtg aacctaaaac aggtaaaatt 540

aaaaaaatca tgtctggtat taaatctcaa cttcaagcta cacctacagt tattcaagaa 600

ctttctcaaa cagtattcaa agatattgga cgtaaccctg atcatgtttc ttctttccaa 660

gctccttgtt ctattcttaa ccaacgtgtt tcttcttctc gtcgtttcgc agcacaatct 720

ttcgaccttg atcgtttccg taacattgct aaatctctta acgttactat taacgatgtt 780

gtacttgctg tatgttctgg tgcattacgt gcttatctta tgtctcataa ctctcttcct 840

tctaaaccat taattgctat ggttccagct tctattcgca acgacgattc tgatgtttct 900

aaccgtatta ctatgattct tgcaaacctt gcaactcaca aagatgatcc tttacaacgt 960

cttgaaatta tccgccgttc tgttcaaaac tctaaacaac gcttcaaacg tatgacttct 1020

gatcaaattc ttaactattc tgctgttgta tatggtcctg caggacttaa catcatttct 1080

ggtatgatgc caaaacgcca agcattcaac cttgttattt ctaacgttcc tggtccacgt 1140

gaaccacttt actggaacgg tgctaaactt gatgcacttt acccagcttc tattgtatta 1200

gacggtcaag cacttaacat tacaatgact tcttatttag ataaacttga agttggtctt 1260

attgcatgtc gtaacgcact tccacgtatg caaaacttac ttacacattt agaagaagaa 1320

attcaacttt tcgaaggtgt aattgcaaaa caagaagata ttaaaacagc taactaa 1377

<210> 9
<211> 37
<212> DNA
<213> Artificial Sequence

<220>
<223> Direct primer of gene LcfA

<400> 9
ggagcttcta gatctaaagg ggaggtttta tgcagtc 37

<210> 10
<211> 36
<212> DNA
<213> Artificial Sequence

<220>
<223> Reverse primer of gene LcfA

<400> 10
ttgcttgcat gctcatggat ccttcctata gaaatg 36

<210> 11
<211> 36
<212> DNA
<213> Artificial Sequence

<220>
<223> Direct primer of gene yhfL

<400> 11

ggcttcacta atctagatct attcattcac ttaagg                                36

<210>  12
<211>  35
<212>  DNA
<213>  Artificial Sequence

<220>
<223>  Reverse primer of gene yhfL

<400>  12
aacgaagtcg acggatccgc tttttcattt tattg                                 35

<210>  13
<211>  33
<212>  DNA
<213>  Artificial Sequence

<220>
<223>  Direct primer of operon accDA

<400>  13
agcgagtcta gatgttgcgg aaggcattat gac                                   33

<210>  14
<211>  38
<212>  DNA
<213>  Artificial Sequence

<220>
<223>  Reverse primer of operon accDA

<400>  14
aaaagcatgc cgtcgactta gtttaccccg atatattg                              38

<210>  15
<211>  37
<212>  DNA
<213>  Artificial Sequence

<220>
<223>  Direct primer of operon accBC

<400>  15
tataaatcta gaaatgtcga ccataggagt gcgattg                               37

<210>  16
<211>  32
<212>  DNA
<213>  Artificial Sequence

<220>
<223>  Reverse primer of operon accBC

<400>  16
ttcatcgcat gcacctccgt aaaaattatg ag                                    32

<210>  17
<211>  555
<212>  DNA
<213>  Artificial Sequence

<220>
<223>  Synthetic mutated sequence of gene ´tesA

<400>  17
atggcggcgg atacactttt gattttagga gatagcctgt cagcgggtta ccggatgtca      60

gcgtctgcgg cgtggcctgc gttgttaaat gataaatggc aatctaaaac atccgttgtg     120

aacgcatcaa ttagcggaga cacgagccaa cagggtttag cacgtttgcc ggcgctgctt     180

aaacaacatc agcctcggtg ggtcctggta gaacttggcg gaaatgatgg tttaagaggc     240

tttcaaccgc aacagacaga acagacgtta cgccaaattt tgcaggacgt taaagcagcg     300

aacgctgaac cgttattgat gcaaatccgt ctgcctgcca attatggcag acgctacaac     360

gaagcatttt cagccattta tccgaaactg gcaaaagaat ttgatgttcc gctgcttccg     420

tttttcatgg aagaagtcta ccttaaaccg caatggatgc aggatgacgg aatccatcct     480

aatcgcgatg cgcagccttt tatcgccgac tggatggcaa aacaattaca gcctctggtg     540

aatcatgact cctaa                                                      555


<210>  18
<211>  1740
<212>  DNA
<213>  Artificial Sequence

<220>
<223>  Synthetic mutated sequence of gene pdc also containing sites
       for restriction enzymes and a ribosome-binding region
       (rbs).


<220>
<221>  misc_feature
<222>  (1)..(6)
<223>  Sequence of restriction enzyme HindIII

<220>
<221>  RBS
<222>  (7)..(21)

<220>
<221>  misc_feature
<222>  (1729)..(1734)
<223>  Sequence of restriction enzyme SalI

<220>
<221>  misc_feature
<222>  (1735)..(1740)
<223>  Sequence of restriction enzyme BamHI

<400>  18
aagcttagga ggaaataact aatgtcttat actgttggta cttatttagc tgaacgtctt      60

gttcaaattg gtcttaaaca tcacttcgca gttgctggtg actacaacct tgttcttctt 120

gacaaccttc ttcttaacaa aaacatggaa caagtttatt gctgtaacga acttaactgc 180

ggtttctctg cagaaggtta tgctcgtgct aaaggtgcag cagcagctgt tgttacttac 240

tctgttggtg ctctttctgc attcgatgct atcggtggtg cttatgcaga aaaccttcct 300

gttatcctta tctctggtgc tcctaacaac aacgaccacg ctgctggtca cgttcttcat 360

cacgctcttg gtaaaactga ctatcactat caacttgaaa tggctaaaaa catcactgct 420

gctgctgaag ctatttatac tcctgaagaa gctcctgcta aaatcgatca cgttattaaa 480

actgctcttc gtgaaaaaaa acctgtttat cttgaaatcg cttgcaacat tgcttctatg 540

ccttgcgctg ctcctggacc tgcatctgca cttttcaacg acgaagcatc tgacgaagca 600

tctcttaacg cagctgttga agaaactctt aaattcatcg ctaaccgcga caaagttgct 660

gttcttgttg gttctaaact tcgcgcagct ggtgctgaag aagctgctgt taaattcgct 720

gatgctcttg gtggtgcagt tgctactatg gctgctgcaa aatctttctt cccagaagaa 780

aaccctcatt acatcggtac ttcttggggt gaagtttctt atcctggtgt tgaaaaaact 840

atgaaagaag ctgatgctgt tatcgctctt gctcctgttt tcaacgacta ctctactact 900

ggttggactg atattcctga tcctaaaaaa cttgttcttg ctgaacctcg ttctgttgtt 960

gttaacggta ttcgcttccc ttctgttcat cttaaagact atcttactcg tcttgctcaa 1020

aaagtttcta aaaaaactgg tgctcttgac ttcttcaaat ctcttaacgc aggtgaactt 1080

aaaaaagctg ctcctgctga tccttctgct cctcttgtta acgcagaaat cgctcgtcaa 1140

gttgaagctc ttcttactcc taacactact gttattgctg aaactggtga ctcttggttc 1200

aacgctcaac gcatgaaact tcctaacggt gctcgcgttg aatatgaaat gcaatggggt 1260

cacattggtt ggtctgttcc tgctgctttc ggttatgctg ttggtgctcc tgaacgtcgc 1320

aacatcctta tggttggtga tggttctttc caacttactg ctcaagaagt tgctcaaatg 1380

gttcgcctta aacttcctgt tatcatcttc cttatcaaca actatggtta cactatcgaa 1440

gttatgatcc atgatggtcc ttacaacaac atcaaaaact gggattatgc tggtcttatg 1500

gaagttttca acggtaacgg tggttatgac tctggtgctg gtaaaggtct taaagctaaa 1560

actggtggtg aacttgcaga agctatcaaa gttgctcttg caaacactga cggtccaact 1620

cttatcgaat gcttcatcgg tcgtgaagac tgcactgaag aacttgttaa atggggtaaa 1680

cgcgttgctg ctgctaactc tcgtaaacct gttaacaaac ttctttaggt cgacggatcc 1740

<210> 19
<211> 1185
<212> DNA
<213> Artificial Sequence

<220>

<223>  Synthetic mutated sequence of gene adhB also containing sites
       for restriction enzymes and a ribosome-binding region
       (rbs).

<220>
<221>  misc_feature
<222>  (1)..(6)
<223>  Sequence of restriction enzyme HindIII

<220>
<221>  misc_feature
<222>  (7)..(12)
<223> Sequence of restriction enzyme SalI

<220>
<221>  RBS
<222>  (13)..(27)

<220>
<221>  misc_feature
<222>  (1180)..(1185)
<223>  Sequence of restriction enzyme BamHI

<400>  19
aagcttgtcg acaggaggaa ataactaatg gcttcttcta ctttctatat tcctttcgtt      60

aacgaaatgg gtgaaggttc tcttgaaaaa gcaatcaaag accttaacgg ttctggtttc     120

aaaaacgctc ttatcgtttc tgatgctttc atgaacaaat ctggtgttgt taaacaagtt     180

gctgaccttc ttaaagcaca aggtattaac tctgctgttt atgatggtgt tatgcctaac     240

cctactgtta ctgcagttct tgaaggtctt aaaatcctta aagataacaa ctctgacttc     300

gttatctctc ttggtggtgg ttctcctcat gactgcgcta aagctatcgc tcttgttgca     360

actaacggtg gtgaagttaa agactacgaa ggtatcgaca aatctaaaaa acctgctctt     420

cctcttatgt ctatcaacac tactgctggt actgcttctg aaatgactcg tttctgcatc     480

atcactgatg aagttcgtca cgttaaaatg gctattgttg accgtcacgt tactcctatg     540

gtttctgtta acgatcctct tcttatggtt ggtatgccaa aaggtcttac tgctgctact     600

ggtatggatg ctcttactca cgcattcgaa gcatattctt ctactgcagc tactcctatc     660

actgatgctt gcgctcttaa agctgcttct atgatcgcta aaaaccttaa aactgcttgc     720

gacaacggta aagatatgcc agctcgtgaa gctatggctt atgctcaatt ccttgctggt     780

atggctttca acaacgcttc tcttggttat gttcatgcta tggctcacca acttggtggt     840

tactacaacc ttcctcatgg tgtttgcaac gctgttcttc ttcctcatgt tcttgcttat     900

aacgcttctg ttgttgctgg tcgtcttaaa gacgttggtg ttgctatggg tcttgatatc     960

gctaaccttg gtgataaaga aggtgcagaa gctactattc aagctgttcg cgatcttgct    1020

gcttctattg gtattccagc aaaccttact gaacttggtg ctaaaaaaga agatgttcct    1080

cttcttgctg accacgctct taaagatgct tgtgctctta ctaaccctcg tcaaggtgat    1140

caaaaagaag ttgaagaact tttcctttct gctttctaag gatcc                    1185

<210>  20
<211>  588
<212>  DNA
<213>  Artificial Sequence

<220>
<223>  Synthetic mutated sequence of gene ´tesA also containing
       sites for restriction enzymes and a ribosome-binding region
       (rbs).

<220>
<221>  misc_feature
<222>  (1)..(6)
<223>  Sequence of restriction enzyme HindIII

<220>
<221>  misc_feature
<222>  (7)..(12)
<223>  Sequence of restriction enzyme BgIII

<220>
<221>  RBS
<222>  (13)..(27)

<220>
<221>  misc_feature
<222>  (583)..(588)
<223>  Sequence of restriction enzyme BamHI

<400>  20
aagcttagat ctaggaggaa ataactaatg gcagctgaca ctttacttat tcttggtgat      60

tctctttctg ctggttatcg tatgtctgct tctgctgctt ggcctgctct tcttaacgat     120

aaatggcaat ctaaaacttc tgtagttaac gcttctatct ctggtgacac ttctcaacaa     180

ggacttgctc gccttcctgc tcttcttaaa caacatcaac ctcgttgggt tcttgttgaa     240

cttggtggta acgacggtct tcgtggtttc caaccacaac aaactgaaca aactcttcgc     300

caaattcttc aagatgttaa agctgctaac gctgaaccac ttttaatgca aatccgtctt     360

cctgcaaact atggtcgccg ttataacgaa gcattctctg ctatttaccc taaacttgct     420

aaagaattcg atgttcctct tcttcctttc ttcatggaag aagtttacct taaaccacaa     480

tggatgcaag atgacggtat tcatcctaac cgcgacgctc aacctttcat tgctgactgg     540

atggctaaac aacttcaacc tttagtaaac catgactctt aaggatcc                  588

<210>  21
<211>  581
<212>  DNA
<213>  Artificial Sequence

<220>
<223>  Synthetic mutated sequence of gene ´tesA also containing
       sites for restriction enzymes and a ribosome-binding region

(rbs).

<220>
<221> misc_feature
<222> (1)..(5)
<223> Sequence of restriction enzyme XbaI

<220>
<221> RBS
<222> (6)..(20)

<220>
<221> misc_feature
<222> (576)..(581)
<223> Sequence of restriction enzyme BamHI

<400> 21
tctagaggag gtgaattgct atggcggcgg atacactttt gattttagga gatagcctgt 60

cagcgggtta ccggatgtca gcgtctgcgg cgtggcctgc gttgttaaat gataaatggc 120

aatctaaaac atccgttgtg aacgcatcaa ttagcggaga cacgagccaa cagggtttag 180

cacgtttgcc ggcgctgctt aaacaacatc agcctcggtg ggtcctggta gaacttggcg 240

gaaatgatgg tttaagaggc tttcaaccgc aacagacaga acagacgtta cgccaaattt 300

tgcaggacgt taaagcagcg aacgctgaac cgttattgat gcaaatccgt ctgcctgcca 360

attatggcag acgctacaac gaagcatttt cagccattta tccgaaactg gcaaaagaat 420

ttgatgttcc gctgcttccg tttttcatgg aagaagtcta ccttaaaccg caatggatgc 480

aggatgacgg aatccatcct aatcgcgatg cgcagccttt tatcgccgac tggatggcaa 540

aacaattaca gcctctggtg aatcatgact cctaaggatc c 581

<210> 22
<211> 1410
<212> DNA
<213> Artificial Sequence

<220>
<223> Synthetic mutated sequence of gene atf1 also containing
sites for restriction enzymes and a ribosome binding
region (rbs).

<220>
<221> misc_feature
<222> (1)..(6)
<223> Sequence of restriction enzyme HindIII

<220>
<221> misc_feature
<222> (7)..(12)
<223> Sequence of restriction enzyme BgIII

<220>
<221> RBS
<222> (13)..(27)

<220>
<221>  misc_feature
<222>  (1405)..(1410)
<223>  Sequence of restriction enzyme BamHI

<400>  22
aagcttagat ctaggaggaa ataactaatg cgcccattac atcctattga tttcatcttc      60

ctttctcttg aaaaacgtca acaacctatg catgtaggtg gtttattcct tttccaaatt     120

cctgataacg ctccagacac tttcattcaa gaccttgtta acgatatccg tatctctaaa     180

tctatccctg ttccaccatt caacaacaaa cttaacggtc ttttctggga tgaagatgaa     240

gaattcgatt tagatcatca tttccgtcat attgcacttc ctcatcctgg tcgtattcgt     300

gaacttctta tttatatttc tcaagaacac tctactcttc ttgatcgtgc aaaacctctt     360

tggacttgca acattattga aggaattgaa ggtaaccgtt tcgctatgta cttcaaaatt     420

caccatgcta tggttgatgg tgttgctggt atgcgtttaa ttgaaaaatc tctttctcat     480

gatgtaacag aaaaatctat cgttccacct tggtgtgttg aaggaaaacg tgcaaaacgc     540

ttacgtgaac ctaaaacagg taaaattaaa aaaatcatgt ctggtattaa atctcaactt     600

caagctacac ctacagttat tcaagaactt tctcaaacag tattcaaaga tattggacgt     660

aaccctgatc atgtttcttc tttccaagct ccttgttcta ttcttaacca acgtgtttct     720

tcttctcgtc gtttcgcagc acaatctttc gaccttgatc gtttccgtaa cattgctaaa     780

tctcttaacg ttactattaa cgatgttgta cttgctgtat gttctggtgc attacgtgct     840

tatcttatgt ctcataactc tcttccttct aaaccattaa ttgctatggt tccagcttct     900

attcgcaacg acgattctga tgtttctaac cgtattacta tgattcttgc aaaccttgca     960

actcacaaag atgatccttt acaacgtctt gaaattatcc gccgttctgt tcaaaactct    1020

aaacaacgct tcaaacgtat gacttctgat caaattctta actattctgc tgttgtatat    1080

ggtcctgcag gacttaacat catttctggt atgatgccaa aacgccaagc attcaacctt    1140

gttatttcta acgttcctgg tccacgtgaa ccactttact ggaacggtgc taaacttgat    1200

gcactttacc cagcttctat tgtattagac ggtcaagcac ttaacattac aatgacttct    1260

tatttagata aacttgaagt tggtcttatt gcatgtcgta acgcacttcc acgtatgcaa    1320

aacttactta cacatttaga agaagaaatt caacttttcg aaggtgtaat tgcaaaacaa    1380

gaagatatta aaacagctaa ctaaggatcc                                     1410

## Description

### FIELD OF THE INVENTION

The present invention discloses a process for the synthesis of fatty acid ethyl esters (FAEEs), also known as biodiesel, from glycerin or glycerol. Therefore, the present invention may be included within the field of industrial biorefineries.

### STATE OF THE ART

Raw glycerin, or glycerol, is a by-product of the biodiesel manufacturing process in industrial biorefineries. The main function of biodiesel is its use as a fuel, to replace petroleum-derived fuels, due to the inherent problems generated by the use of petroleum-derived fuels, in terms of pollution, the greenhouse effect and global warming; consequently, there is a need for a renewable petroleum source that, moreover, may be produced in an inexpensive manner.

In this regard, biodiesel may be used in most diesel internal combustion engines, either in pure form, what is known as "clean biodiesel", or as a mixture, at any concentration, with petroleum-derived diesel fuel. Biodiesel offers advantages with respect to petroleum-derived diesel fuels, including the reduction of gas emissions during combustion and the fact that it is capable of maintaining an equilibrated carbon dioxide cycle, since it is based on the use of renewable biological materials, it is biodegradable and offers greater safety due to its high flash point and low flammability. Another advantage is the lower wear of engines, thanks to its good lubricating properties.

One of the uses of the glycerin obtained as a by-product of biodiesel production is the obtainment of biodiesel itself, by means of an alternative process that comprises using said glycerin (glycerol) as a substrate in bacterial fermentation processes.

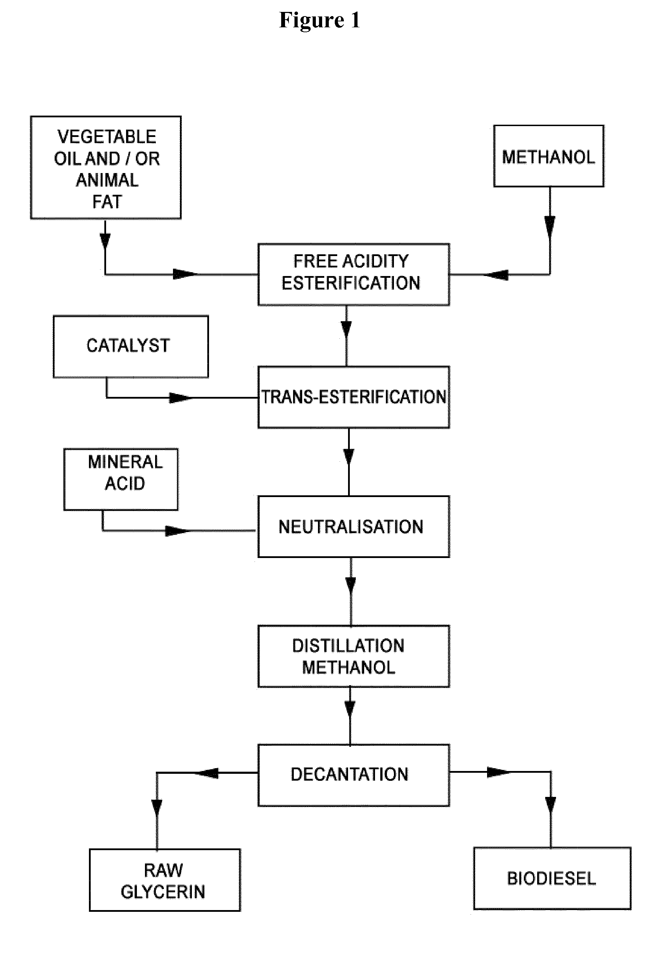

Biodiesel is primarily obtained from vegetable oils or animal fats, by means of industrial esterification and transesterification processes (Figure 1) (Karmakar A., et al. 2010). The glycerin formed in the process of obtaining industrial biodiesel is an industrial waste that has to be disposed of due to its low cost in recent years.

Currently, one of the main concerns in the field of industrial biorefineries is how to provide an outlet for this by-product that is causing a great negative impact to said companies in economic and environmental terms. A vegetable-origin biodiesel plant that generates about 250,000 tonnes of biodiesel/year produces 10% of glycerin, i.e. about 25,000 tonnes/year of glycerin. Disposing of said by-product involves huge expenses, such as, for example, its transport, in order to eventually commercialise it. In view of this situation, the development of biotechnological processes aimed at converting raw glycerin into high-added-value products is a "need". These biotechnological processes could be incorporated into already-existing biodiesel plants, where the glycerin generated as a by-product of the manufacturing of said biodiesel would be processed *in situ,* or result in biorefineries that integrate multiple processes, based on a concept analogous to that used for industrial refineries, which produce multiple fuels and products from petroleum.

For years, different bacterial strains, primarily of the species *Escherichia coli,* have been used for the production of FAEEs, and significant advances have been made in the understanding of the lipid synthesis pathway and its regulation in bacteria. In the past, said regulation has been presented as the main limiting factor for the use of bacteria or plants as oil or fuel "factories". Vaneechoutte M. et al. disclose that, in order to produce FAEEs from *E. coli,* it is necessary to esterify the ethanol produced in the metabolism of the synthesis of said FAEEs with Coenzyme-A-bound acyl residues, by the overexpression of the triacylglycerol and wax synthase enzyme (Atf1) of *Acinetobacter baylyi* (Atsumi S, et al. 2008). Said strain of transformed *E. coli* is capable of producing FAEE concentrations of 1.3 g/l with a production yield of 0.018 g/(1/h) in the presence of glucose and oleic acid (Atsumi S, et al. 2008). In this regard, Kalscheuer R. et al. demonstrated that FAEEs may be produced from the heterologous expression of the pyruvate decarboxylase (Pdc) and alcohol dehydrogenase (AdhB) enzymes of *Zymomonas mobilis* and the non-specific synthase (Atf1) of the *Acinetobacter baylyi ADP1* strain in *E. coli* (Kalscheuer R. et al 2008). Said synthesis of ethanol was combined with its subsequent esterification with the fatty acid acyl residues of Coenzyme A when the bacteria were cultured under aerobic conditions, in the presence of glucose and oleic acid.

International patent application WO2011/038134 also discloses the production of biodiesel from genetically-modified bacterial organisms. Said international application shows examples of *E. coli* bacteria capable of producing biodiesel thanks to the expression or overexpression of a gene that encodes a thioesterase, a gene that encodes an Acyl-CoA synthetase and a gene that encodes an ester synthase. The biofuel generated by the bacteria described in said international application is primarily composed of linear fatty acids with an even number of carbon atoms, on average, 50% thereof, with one unsaturated bond.

Other authors have also disclosed the production of biofuels from the conversion of glycerol into ethanol by means of different genetically-modified strains of *E.coli,* designed to produce said biofuels (Shams Yazdani S. et al. 2008; da Silva GP. et al. 2009; Choi WJ. 2008). Other bacterial species have also been genetically modified for the production of said biofuel by means of the transformation of glycerol into ethanol. For example, mutant strains of *Klebsiella pneumoniae,* obtained by y irradiation and transformed with the *pdc* and *adhII* genes obtained from Z. *mobilis* and which overexpress the pdc and adh enzymes, respectively, show a production of ethanol from glycerol of 25 g/l (Oh BR. et al., 2011). Thus far, the main bacterial strains used for the synthesis of FAEEs from glycerol have been pathogenic (or potentially pathogenic) strains, such as, for example, Z. *mobilis* and *E. coli,* as discussed above.

The purpose of obtaining said bacterial strains, capable of synthesising FAEEs from glycerol, is that said strains are capable of secreting the synthesised FAEEs to the exterior. In this regard, as previously discussed, the main bacterial strains used thus far in the state of the art to obtain FAEEs are primarily pathogenic bacteria (*Z. mobilis* and *E. coli);* for this reason, once the FAEEs have been synthesised, and given that the bacterial remnants in the media are toxic, they may represent a hazardous waste disposal problem, since, for example, *E. coli* is responsible for the enteric pollution of drinking water (coliform bacteria).

The present invention discloses a method for producing biodiesel (FAEE) from glycerin as the carbon source, by means of the transformation of non-pathogenic bacteria of the genus *Bacillus* with different heterologous synthetic mutated genes involved in the synthesis of said FAEEs. Bacteria of the genus *Bacillus* are approved for consumption in humans and are used for large-scale production in industry, such as in biofactories designed for the production of different enzymes and proteins (Westers et al. 2004). More specifically, the invention discloses bacteria of the species *Bacillus subtilis* transformed with heterologous synthetic mutated genes specifically designed to increase their expression in the bacteria of said species. In this regard, the genes of the pyruvate decarboxylase (*pdc*) (SEQ ID NO: 1) and alcohol dehydrogenase (*adhB*) (SEQ ID NO: 3) enzymes pertaining to *Zymomonas mobilis,* and the active portion of the thioesterase A (*'tesA*) enzyme (SEQ ID NO: 5) of *Escherichia coli* and the triacylglycerol and wax synthase *(atf1*) enzyme (SEQ ID NO: 7) of *Acinetobacter sp.* ADP1 have been isolated. From the sequences of said genes, the corresponding synthetic mutated genes of the *pdc* (SEQ ID NO: 2), *adhB* (SEQ ID NO: 4), *'tesA* (SEQ ID NO: 6 or SEQ ID NO: 17) and *atf1* (SEQ ID NO: 8) enzymes have been obtained, with which the *B. subtilis* bacteria described in the present invention have been transformed. Moreover, in order to clone said genes in an expression plasmid, such that they are subsequently expressed *B. subtilis,* sequences that encode specific restriction enzyme sites and the ribosome-binding sequence (rbs) were added to the ends of the synthetic mutated sequences, in order to favour the expression thereof in *B. subtilis,* leading to sequences SEQ ID NO: 18, for the *pdc* gene, SEQ ID NO: 19, for the *adhB* gene, SEQ ID NO: 20 or 21, respectively, for the *'tesA* gene, and SEQ ID NO: 22, for the *atf1* gene, respectively. Moreover, in order to increase the yield of biodiesel production by means of the bacteria described in the present invention, at least one of the genes of *B. subtilis* that encode the acyl-CoA synthetase *(lcfA, yhfL* and *yhfT*) enzymes (E.C. 6.2.1.3) and the four subunits of the acetyl-CoA carboxylase (*accDABC*) enzyme (E.C. 6.4.1.2) may be overexpressed therein. Moreover, it is worth noting that bacteria of the genus *B. subtilis* are capable of growing using glycerol as the only source of carbon and energy.

At the same time, use of the strains described in the invention for the synthesis of FAEEs from glycerin has the following advantages:
- In general, at the process level (ability to consume substrates, maximum achievable cell mass, yields, etc.), *B. subtilis* is as efficient as or more efficient than *E*. *coli* (Westers et al., 2004).
- *B. subtilis* is not pathogenic and, in fact, it is used as probiotic food for animals designed for human consumption. Moreover, the FAEEs would be secreted outside the cell and, instead of creating a hazardous waste disposal problem, the remnant bacteria would have added value, whereas *E. coli* or K. *pneumoniae* could not used for that purpose, since they are pathogenic.
- The fatty acid synthesis pathway in *B. subtilis* makes it possible to manipulate, in a relatively simple manner, modifications in the omega-terminal methylation of FAEEs, which may lead to the production of biofuels with different or better properties than the linear FAEEs habitually synthesised, such as, for example, the production of fully saturated branched fatty acids.
- *B. subtilis* efficiently secretes hydrolytic enzymes, such as glucanases, amylases, lipases, cellulases, etc., which makes it possible to adapt said bacteria for the degradation of other cheap compounds which are not directly assimilable by *E. coli* or other organisms as the carbon sources.
- The genetic modifications proposed in the present invention are very stable in *B*. *subtilis,* since the simple integration of said modifications at different sites in its genome makes it unnecessary to use replicating plasmids.

Therefore, in order to solve the problem of excess glycerin arising from biodiesel production processes, and jointly with the need to obtain a biofuel that does not have, as mentioned above, as many disadvantages as petroleum-derived fuels, the present invention discloses strains of the species *B. subtilis* capable of increasing the production of biodiesel from the by-product, glycerin, as the carbon source, upon being transformed with heterologous synthetic mutated genes capable of inducing a more efficient expression in *B. subtilis,* obtained from the following genes: *pdc* (SEQ ID NO: 1) and *adhB* (SEQ ID NO: 3), originating from Z. *mobilis, 'tesA* (SEQ ID NO:5), originating from *E. coli,* and *atf1* (SEQ ID NO: 7), originating from *Acinetobacter sp.* ADP1. Furthermore, the strains of the present invention overexpress at least one of the genes of the ACC (acetyl-CoA carboxylase) (E.C. 6.4.1.2) and acyl-CoA synthetase (E.C. 6.2.1.3) enzymatic complexes. In turn, the present invention discloses the use of said strains for the synthesis of biodiesel and a method for obtaining biodiesel from glycerin as the carbon source, based on the use of said strains.

### DESCRIPTION OF THE INVENTION

### Brief description of the invention

In order to solve the problems described above, the present invention discloses bacterial strains of the species *B. subtilis,* preferably strains CECT 7968, CECT 7969 and NCIMB 42026, which have been transformed with heterologous synthetic mutated genes optimised for a more efficient expression in *B. subtilis,* and which, moreover, in order to increase the yield of biodiesel production using said bacterial strains, overexpress at least one of the bacterial genes that encode the ACC (E.C. 6.4.1.2) and acyl-CoA synthetase (E.C. 6.2.1.3) enzymatic complexes, leading to a greater production of biodiesel from its own by-product, glycerin, as the carbon source. Said genes encode enzymes involved in bacterial lipid metabolism, especially the synthesis of lipids from glycerin as the carbon source.

On the other hand, the heterologous synthetic mutated genes with which the *B. subtilis* bacteria of the present invention are transformed are: *pdc* (SEQ ID NO: 2 or SEQ ID NO: 18) and *adhB* (SEQ ID NO: 4 or SEQ ID NO: 19), obtained from the *pdc* (SEQ ID NO: 1) and *adhB* (SEQ ID NO: 3) genes of *Z*. *mobilis, 'tesA* (SEQ ID NO: 6 or SEQ ID NO: 17 or SEQ ID NOs: 20-21), obtained from the *'tesA* (SEQ ID NO: 5) gene of *E. coli,* and *atf1* (SEQ ID NO: 8 or SEQ ID NO: 22), obtained from the *atf1* (SEQ ID NO: 7) gene of *Acinetobacter sp.* ADP1. Furthermore, the bacterial strains described in the present invention overexpress at least one of the genes that encode the ACC (acetyl-CoA carboxylase) (E.C. 6.4.1.2) and acyl-CoA synthetase (E.C. 6.2.1.3) enzymatic complexes.

Another object described in the present invention relates to the use of the strains described above, preferably strains CECT 7968, CECT 7969 and NCIMB 42026, for the production of biodiesel from glycerin as the carbon source.

Another object of the present invention relates to a process for the production of biodiesel from glycerin as the carbon source, using the bacterial strains described in the present invention (CECT 7968, CECT 7969 and NCIMB 42026). The composition of the biodiesel thus produced may be used as a diesel fuel by itself, or mixed with petroleum diesel in the habitual proportions, which leads to cleaner emission profiles.

Another aspect of the present invention relates to a fuel composition, including, for example, a diesel fuel composition, which comprises a fatty ester produced by a method or a genetically modified microorganism such as those described in the present invention. In certain embodiments, the fuel composition further comprises one or more adequate fuel additives. The fuel obtained by means of the process described in the present invention using strains CECT 7968, CECT 7969 and NCIMB 42026 is preferably composed of fully saturated branched fatty acid ethyl esters, with an odd number of carbon atoms, said fatty acid ethyl esters preferably being: iso-C15 (13-methyltetradecanoic acid), anteiso-C15:0 (12-methyltetradecanoic acid), n-C15:0 (n-pentadecanoic acid); iso-C17:0 (15-methylhexadecanoic acid), anteiso-C17:0 (14-methylhexadecanoic acid) and n-C17:0 (n-heptadecanoic acid). The fuel obtained by means of the process described above may also be composed of branched fatty acid ethyl esters with an even number of carbon atoms (although this type of fatty acids are a minority), said ethyl esters preferably being: iso-C16:0 (14-methylpentadecanoic acid), n-C16:0 (n-hexadecanoic acid), iso-C18:0 (16-methylheptadecanoic acid) and n-C18:0 (n-octadecanoic acid), as described, for both cases (ethyl esters with an odd or even number of carbon atoms), in Example 3.

Except as otherwise specified, all the technical and scientific terms used in the present document have the same meaning as that commonly understood by persons skilled in the art whereto this invention pertains.

As used herein, the term "biodiesel" is a biofuel that may be a substitute for petroleum-derived diesel fuel. Biodiesel may be used in diesel internal combustion engines, either in pure form, what is known as "clean", or as a mixture, at any concentration, with petroleum-derived diesel. In one embodiment, the biodiesel may include esters or hydrocarbons, such as aldehydes, alkanes or alkenes. The term "biofuel" refers to any type of fuel derived from biomass or biological sources in general. Biofuels may substitute for petroleum-based fuels. For example, biofuels include all transport fuels (for example, petrol, diesel, jet fuel, etc.), heating fuels and fuels designed to generate electricity. Biofuels are a renewable energy source.

The term "carbon source" refers to a substrate or compound that is adequate to be used as a supplier of carbon atoms for the growth of prokaryotic or eukaryotic cells. Carbon sources may be of several types, including, without being limited thereto, organic fatty acids, such as, for example, succinate, lactate and acetate, polymers, carbohydrates, acids, alcohols, aldehydes, ketones, amino acids, peptides and gases (for example, CO and CO₂). They also include, for example, various monosaccharides, such as glucose, fructose, mannose and galactose; oligosaccharides, such as fructo-oligosaccharides and galacto-oligosaccharides; polysaccharides, such as xylose and arabinose; disaccharides, such as sucrose, maltose and turanose; cellulosic material, such as sodium methyl cellulose and carboxymethyl cellulose; saturated or unsaturated fatty acid esters; alcohols, such as glycerol, methanol, ethanol, propanol; or mixtures thereof. The carbon source may also be a product of photosynthesis, including, without being limited thereto, glucose. For purposes of the present invention, a preferred carbon source is glycerol or glycerin.

For purposes of the present invention, the term "promoter element", "promoter", or "promoter sequence" refers to a DNA sequence that acts as a switch that activates the expression of a gene, thanks to its interaction with RNA polymerase. If the gene is activated, it is said to be transcribed or participate in the transcription. The transcription involves the synthesis of gene mRNA. Therefore, a promoter acts as a transcriptional regulatory element and also provides a site for gene transcription initiation in mRNA. Depending on the expression level, promoters may be divided into weak or strong promoters. A strong promoter is one that, due to its high affinity for RNA polymerase, allows for the formation of a large quantity of RNA, which, ultimately, will translate into proteins (when its expression is permitted). On the contrary, a weak promoter has a low affinity for RNA polymerase, and, consequently, under its control, gene expression levels are much lower. Depending on the control mechanism, promoters may be classified into constitutive or regulatable promoters. A promoter is said to be constitutive when it allows for the expression of the gene or genes under its control to manifest itself continuously. On the contrary, regulatable promoters only allow for the expression of the genes that they control when their product is essential. They may be inducible, when they only allow for the expression of the gene or genes that they regulate following a given stimulus (in the presence of inducing conditions), and repressible, when the expression of the genes under their control is continuous and only stops following a given stimulus or under certain conditions (under repressing conditions).

For purposes of the present invention, the terms "glycerin" and "glycerol" are synonymous and are used interchangeably.

For purposes of the present invention, the term "regulon" is described as a group of genes regulated by the same transcription factor. The regulated genes may grouped, as in the case of operons, or dispersed throughout the genome.

For purposes of the present invention, the term "operon" is defined as a functional gene unit formed by a group or complex of genes that are transcribed from the same promoter, and the expression thereof is co-ordinated by the same systems.

For purposes of the present invention, the term "heterologous", in relation to genes, proteins, tissues, cells, etc., is defined as pertaining to an individual from a genus or species different from that of the individual wherefrom it is isolated.

For purposes of the present invention, the term "host cell or organism" refers to a cell or organism that may be genetically modified to express, overexpress or underexpress specific selected genes. Non-limiting examples of host cells or organisms include vegetable, animal, human, bacterial or fungal cells. The preferred host cells in the present invention are bacterial cells.

For purposes of the present invention, the terms "to overexpress" or "overexpression" refer to a greater expression level or higher concentration of a nucleic acid, polypeptide, protein, enzyme, carbohydrate, fat, hydrocarbon, etc., in a given cell, as compared to the expression level or concentration of said nucleic acid, polypeptide, protein, enzyme, carbohydrate, fat, hydrocarbon, in the same type of cell belonging to a wild-type phenotype. For example, a polypeptide may be "overexpressed" in a recombinant host cell when the polypeptide is present at a higher concentration than in the non-recombinant cell of the same species.

As used herein, the term "recombinant polypeptide" refers to a polypeptide that is produced by means of recombinant DNA techniques, where, in general, the DNA or RNA that encodes the expressed polypeptide is inserted into an adequate expression vector, which, in turn, is used to transform a host cell in order to produce the polypeptide or RNA.

The term "mutated genes" refers to the introduction of mutations in the coding region of genes in order to increase the production of proteins. The criteria used to perform the mutations are described in Example 2 of the present invention.

For purposes of the present invention, the term "synthetic" is defined as referring to gene or protein sequences obtained artificially by means of genetic engineering processes.

Throughout the entire description of the present patent, the terms "comprises" or "which comprises" mean the presence of the characteristics, elements, integers, steps or components, especially the DNA sequences or protein sequences, mentioned in the claims, but do not exclude the presence or addition of one or more additional characteristics, elements, integers, steps, components, sequences or groups thereof. The terms "comprises" or "which comprises" are also intended to include embodiments covered by the terms "which is essentially composed of" and "which is composed of". Likewise, the term "is essentially composed of" is intended to include embodiments comprised by the term "which is composed of".

The term "variant" used in the present invention is understood to mean a nucleotide sequence of the genes described in the present invention or even an amino acid sequence of a protein or polypeptide, encoded by the aforementioned nucleotide sequences, modified in one or more of the nucleotides or even the amino acids thereof. For purposes of the present invention, the term "variant" preferably refers to a modification of one or more nucleotides in a given gene sequence. The variant may have "conservative" changes, wherein the substituted nucleotide or amino acid has similar structural or chemical properties to those of the substituted nucleotide or amino acid. The variant may also have "non-conservative" changes or a deletion and/or insertion of one or more nucleotides or amino acids. For purposes of the present invention, the variant is preferably a conservative variant. For purposes of the present invention, a "functional variant" is understood to be a variant that retains the functional capacity of the original nucleotide or amino acid sequence, without modifications or mutations, wherefrom it is derived. For purposes of the present invention, polynucleotide or polypeptide variants are functional variants that present the same function as the original sequences, without modifications or mutations, wherefrom they derive.

For purposes of the present invention, the term "additive" refers to a chemical substance added to a product in order to improve its properties and ensure the correct operation of engines.

### Description of the Figures

Figure 1. Diagram of the synthesis of biodiesel from vegetable oils and/or animal fats.

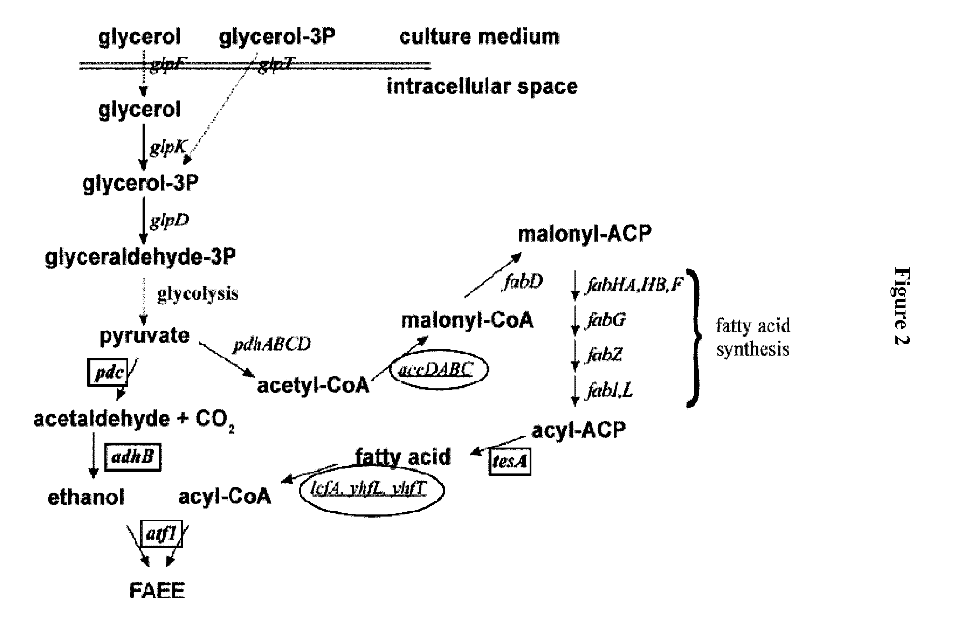

Figure 2. Diagram of the modified metabolic pathways in *B. subtilis* designed to increase the synthesis of biodiesel from glycerin. The genes shown inside squares (*pdc, adhB, 'tesA* and *atf1*) are heterologous synthetic mutated genes, which pertain to species different from *B. subtilis* and which have been obtained synthetically, optimising the use of specific codons to increase the expression of said genes in *B. subtilis.* The genes included in an ellipse (*accDABC, lcfA, yhfL* and y*hfT*) are the genes present in *B. subtilis* which, alternatively, may be overexpresssed in said bacteria, by means of specific promoters designed to increase and enhance the production of biodiesel. *glpF:* glycerol facilitator; *glpT:* glycerol-3P transporter; *glpK:* glycerol kinase; *glpD:* glycerol-3P dehydrogenase; *pdc:* pyruvate decarboxylase; *adhB:* alcohol dehydrogenase; *atfl:* triacylglyceride-wax synthase; *pdh:* pyruvate dehydrogenase; *accDABC:* acetyl-CoA carboxylase; *fabD:* malonyl-CoA:ACP transferase; *lcfA, yhfL, yhfT:* acyl-CoA synthetases.

### Detailed description of the invention

The present invention describes bacterial strains of the species *B. subtilis,* preferably strains CECT 7968, CECT 7969 and NCIMB 42026, capable of producing biodiesel (FAEE) from glycerin as the carbon source. Said bacterial strains of the species *B. subtilis* are transformed with an expression vector that expresses the plasmids pNAKA62 (in strains CECT 7968 and CECT 7969) or pNAKA143 (in strain NCIMB 42026), which carry the heterologous synthetic mutated genes: *pdc* (SEQ ID NO: 2 or SEQ ID NO: 18 or variants thereof) and *adhB* (SEQ ID NO: 4 or SEQ ID NO: 19 or variants thereof), originating from Z. *mobilis, 'tesA* (SEQ ID NO: 6 or SEQ ID NO: 17 or SEQ ID NOs: 20-21 or variants thereof), originating from *E*. *coli,* and *atf1* (SEQ ID NO: 8 or SEQ ID NO: 22 or variants thereof), originating from *Acinetobacter sp.* ADP1, under the control of an inducible promoter. In the case of the present invention, said promoter is the Pspac promoter, which is inducible by isopropyl-β-D-thiogalactoside (IPTG). It is worth noting that, for purposes of the present invention, any inducible or constitutive promoter known in the state of the art may be used to the same end. Moreover, the CECT 7968, CECT 7969 and NCIMB 42026 bacteria described in the present invention overexpress at least one of the genes that encode the ACC (E.C. 6.4.1.2) and Acyl-CoA synthetase (E.C. 6.2.1.3) enzymes, thereby enhancing the synthesis of biodiesel from glycerol, since said genes are expressed in a very low (ACC) or zero quantity (Acyl-CoA synthetase) in the exponential phase of bacterial growth.

The production of biodiesel from glycerin as the carbon source using bacterial strains CECT 7968, CECT 7969 and NCIMB 42026 described in the present invention has been performed by means of different strategies capable of modifying the metabolism thereof, which results in an increase in the synthesis and concentration of fatty acids, jointly with an increase in the synthesis of ethanol. The final result of said increases is the synthesis of biodiesel from the by-product, glycerin, as the carbon source. Bacterial strains CECT 7968, CECT 7969 and NCIMB 42026 present an increase in the synthesis of fatty acids and ethanol thanks to their transformation with the expression vector pDR67, which carries the plasmid pNAKA62 or the plasmid pNAKA143, both capable of expressing, as mentioned above, the four heterologous synthetic mutated genes: SEQ ID NO: 2 or SEQ ID NO: 18 or variants thereof, SEQ ID NO: 4 or SEQ ID NO: 19 or variants thereof, SEQ ID NO: 6 or SEQ ID NO: 17 or SEQ ID NOs: 20-21 or variants thereof, and SEQ ID NO: 8 or SEQ ID NO: 22, or variants thereof, and, moreover, alternatively, by means of their transformation with a vector carrying a plasmid capable of overexpressing at least one of the genes of the ACC (E.C. 6.4.1.2) and/or acyl-CoA synthetase (E.C. 6.2.1.3) enzymatic complexes, said strains increase the yield of production of said biodiesel.

The genes shown in Figure 2 inside squares (*pdc, adhB, 'tesA* and *atf1)* are heterologous synthetic mutated genes, which pertain to species different from *B. subtilis* and which have been obtained synthetically, optimising the use of specific codons for this organism. On the other hand, the genes included in an ellipse (*accDABC, lcfA, yhfL* and *yhfT*) in said Figure 2 are genes that, alternatively, may be overexpresssed in said bacteria by means of specific promoters, such as, for example, IPTG inducible promoters (Pspac or Pspachy) and/or strong constitutive promoters (Pacp), in order to increase and enhance the production of biodiesel. Said promoters facilitate and increase the production of biodiesel thanks to an increase in the expression of the genes of the ACC (E.C. 6.4.1.2) and acyl-CoA synthetase (E.C. 6.2.1.3) complexes, during the exponential growth phase of *B. subtilis,* since their expression is low, in the case of the *accDABC* complex, or null, in the case of the acyl-CoA synthetase complex.

As previously discussed, the increase in the synthesis and concentration of fatty acids produced by the modification of the metabolic pathways of the strains of the invention is a consequence of the expression of the heterologous synthetic mutated gene that encodes the *'tesA* (SEQ ID NO: 6 or SEQ ID NO: 17 or SEQ ID NOs: 20-21 or variants thereof) enzyme, originating from *E. coli.* Said enzyme catalyses the hydrolysis of the thioester bond between acyl groups and the acyl carrier protein (ACP). In this regard, strains of *E. coli* that express the catalytic domain of said enzyme synthesise a large quantity of fatty acids due to the decreased negative feedback that acyl-ACPs exert on fatty acid synthases (FAS). Thus, the strains of the present invention express the mutated catalytic domain of the *'tesA* thioesterase of *E. coli* (SEQ ID NO: 6 or SEQ ID NO: 17 or variants thereof) by optimising the use of specific codons for *B*. *subtilis* bacteria. Moreover, in order to clone said genes in an expression plasmid, such that they are subsequently expressed in *B. subtilis,* sequences that encode specific restriction enzyme sites and the ribosome-binding sequence were added to the ends of SEQ ID NO: 6 and SEQ ID NO: 17, leading to SEQ ID NOs: 20 and 21, respectively. For purposes of the present invention, it is worth noting that any specific sequence of restriction enzymes different from those used in the present invention may be used to the same end. On the other hand, in order to enhance and increase the yield of synthesis of said fatty acids, the bacterial strains described in the present invention may overexpress the ACC enzymatic complex and, in turn, in order to enhance and increase the yield of biodiesel synthesis, they may also overexpress the acyl-CoA ligase enzymatic complex. Both complexes are overexpressed by being transformed with expression vectors carrying plasmids under the control of specific strong constitutive promoters, such as, for example, the *Pacp-accDABC* promoter for the overexpression of acyl-CoA carboxylase (ACC), or inducible promoters, as in the case of the Pspac or Pspachy promoters, which are inducible in the presence of IPTG.

In particular, the fatty acid elongation cycle in *B. subtilis* is performed by fatty acid synthase (FAS). Briefly, the FabF isoform of fatty acid synthase catalyses the condensation of malonyl-ACP with acyl-ACP, to produce elongation of the fatty acid, the FabG isoform reduces the keto acid formed, the FabZ isoform catalyses the dehydration of the hydroxy acid and, finally, the FabI isoform (and occasionally FabL) reduces the double bond formed, generating a new acyl-ACP with two more carbons than at the beginning of the cycle. The FAS enzymatic complex, described in Figure 2, may be overexpressed in the *B. subtilis* strains of the present invention in order to increase the yield of biodiesel synthesis from glycerin as the carbon source. Said overexpression may be performed by means of different strategies. One of said strategies involves the elimination of the global repressor of fatty acid synthesis, FapR (*fapR⁻*)*,* which leads to an FAS activity in *B. subtilis* that is 5 times greater than in strains having said repressor (*fapR⁺*) (Schujman, G.E. et al. 2003). Another strategy that may be used is the overexpression of the ACC enzymatic complex (E.C.6.4.1.2). Said complex catalyses the synthesis of malonyl-CoA from acetyl-CoA. In E. coli, the overexpression of this complex increases FAS activity more than 10-fold, provided that the long-chain acyl-ACPs formed are eliminated, for example, by the expression of the *'tesA* gene (Davis, M.S. et al. 2000).". In order to increase the yield of biodiesel synthesis, the strains of the present invention may overexpress the genes that comprise said ACC enzymatic complex (*accD, accA, accB* and *accC*)*.* To this end, a synthetic operon, *accDABC,* was constructed, which jointly comprises the four genes, whereas in untransformed wild bacteria the operons *accDA* and *accBC* are found separately. The synthetic operon *accDABC* obtained was included in the plasmid pGES468 under the control of a promoter which, as mentioned above, may be strong and constitutive, such as, for example, the P*acp-accDABC* promoter, or inducible, such as Pspac-accDABC, in the presence of, for example, IPTG, although any promoter known in the state of the art may be used. Moreover, for the elimination of the long-chain acyl-ACPs formed by the strains described in the present invention, these strains express the *'tesA* heterologous mutated gene (SEQ ID NO: 6 or SEQ ID NO: 17 or SEQ ID NOs: 20-21 or variants thereof).

The increase in the synthesis of ethanol produced by the strains described in the present invention was obtained by the expression of the heterologous synthetic mutated genes that encode the *pdc* (SEQ ID NO: 2 or SEQ ID NO: 18 or variants thereof) and *adhB* (SEQ ID NO: 4 or SEQ ID NO: 19 or variants thereof) enzymes of Z. *mobilis.* Said enzymes, *pdc* and *adhB,* generate acetaldehyde from acetyl-CoA and, subsequently, ethanol. The *pdc* and *adhB* genes of the bacterial species Z. *mobilis* have been successfully used to transform Gram-negative bacteria, such as, for example, *E. coli* (Kalscheuer, R. et al. 2006). Therefore, through an increase in the concentration of ethanol and acyl-CoAs, and the expression of the *Atf1* (SEQ ID NO: 8 or SEQ ID NO: 22 or variants thereof) heterologous synthetic mutated gene, the strains described in the present invention are capable of esterifying the ethanol synthesised, jointly with the acyl-CoA-bound acyl residues, leading to the production of biodiesel from glycerin, obtained as a by-product of the biodiesel manufacturing process in biorefineries, as the carbon source (see Figure 2).

### Deposit of microorganisms under the Budapest Treaty

The microorganisms used in the present invention were deposited at the Spanish Type Culture Collection (CECT), located at the Research Building of the University of Valencia, Campus Burjassot, Burjassot 46100 (Valencia, Spain), with deposit nos.:
CECT 7968, deposited on 23 June 2011, which comprises the following constructs in its genome: thrC::lacI-Pspachy-lcfA amyE::Pspac-pdc-adh-atf1-'tesA.
CECT 7969, deposited on 23 June 2011, which comprises the following constructs in its genome: thrC::lacI-Pspachy-yhfL amyE::Pspac-pdc-adh-atf1-'tesA.

The other microorganism used in the present invention was deposited on 10 August 2012 at the National Collection of Industrial, Food and Marine Bacteria (NCIMB), located at the Ferguson Building, Craibstone Estate, Bucksburn, Aberdeen, AB21 9YA (United Kingdom), with deposit no. NCIMB 42026, and comprises the following constructs in its genome: thrC::lacI-Pspachy-yhfL amyE::Pspac-pdc-adh-atf1-'tesA.

One of the objects of the present invention relates to a gene construct that comprises at least the genes selected from SEQ ID NO: 2 or SEQ ID NO: 18, SEQ ID NO: 4 or SEQ ID NO: 19, SEQ ID NO: 6 or SEQ ID NO: 17 or SEQ ID NO: 20 or SEQ ID NO: 21 and SEQ ID NO: 8 or SEQ ID NO: 22, or variants thereof, jointly with a promoter.

In a preferred embodiment, the gene construct described in the present invention comprises at least the genes selected from SEQ ID NO: 2, SEQ ID NO: 4, SEQ ID NO: 6 or SEQ ID NO: 17 and SEQ ID NO: 8, or variants thereof, jointly with a promoter.

In another more preferred embodiment, the gene construct described herein comprises at least the genes selected from SEQ ID NO: 18, SEQ ID NO: 19, SEQ ID NO: 20 or SEQ ID NO: 21 and SEQ ID NO: 22, or variants thereof, jointly with a promoter.

In a preferred embodiment of the present invention, the gene construct is characterised in that it further comprises at least one of the nucleotide sequences, or variants thereof, that encode at least one of the genes that form the Acyl-CoA synthetase (E.C.6.2.1.3) and/or ACC (E.C.6.4.1.2) enzymatic complexes, selected from: *lcfA, yhfL, yhfT, accD, accA, accB* or *accC* and/or any combination thereof. In another preferred embodiment of the present invention, the gene construct is characterised in that said genes belong to the bacterial species *B. subtilis.*

In another preferred embodiment, the gene construct described in the present invention is characterised in that the promoter is inducible or constitutive. Preferably, the inducible promoter is the Pspac promoter or the Pspachy promoter, which are inducible by the addition of isopropyl-β-D-thiogalactoside (IPTG) to the culture medium. Preferably, the constitutive promoter is the Pacp promoter. Any other promoter, whether inducible or constitutive, known in the state of the art for the same purpose, may also be used.

In another preferred embodiment, the gene construct described in the present invention is characterised in that it is selected from any of the following: pNAKA62, pNAKA143, pNAKA52, pNAKA53 and/or combinations thereof.

Another object described in the present invention relates to an expression vector characterised in that it comprises sequences SEQ ID NO: 2 or SEQ ID NO: 18, SEQ ID NO: 4 or SEQ ID NO: 19, SEQ ID NO: 6 or SEQ ID NO: 17 or SEQ ID NO: 20 or SEQ ID NO: 21 and SEQ ID NO: 8 or SEQ ID NO: 22 or variants thereof.

In a preferred embodiment, the expression vector described in the present invention comprises at least the genes selected from: SEQ ID NO: 2, SEQ ID NO: 4, SEQ ID NO: 6 or SEQ ID NO: 17 and SEQ ID NO: 8 or variants thereof.

In another more preferred embodiment, the expression vector described in the present invention comprises at least the genes selected from SEQ ID NO: 18, SEQ ID NO: 19, SEQ ID NO: 20 or SEQ ID NO: 21 and SEQ ID NO: 22 or variants thereof.

In a preferred embodiment, the expression vector is characterised in that it further comprises at least one of the nucleotide sequences, or variants thereof, that encode at least one of the genes that form the Acyl-CoA synthetase (E.C.6.2.1.3) and/or ACC (E.C.6.4.1.2) enzymatic complexes, selected from: *lcfA, yhfL, yhfT, accD, accA, accB* or *accC* and/or any combination thereof.

In another preferred embodiment, the expression vector is characterised in that the nucleotide sequences, or variants thereof, that encode at least one of the genes that form the Acyl-CoA synthetase (E.C.6.2.1.3) and/or ACC (E.C.6.4.1.2) enzymatic complexes *(lcfA, yhfL, yhfT, accD, accA, accB* or *accC*) belong to the bacterial species *B. subtilis.*

In another preferred embodiment, the expression vector is characterised in that it comprises at least one of the gene constructs described in the present invention.

In a preferred embodiment, the expression vector is characterised in that it is composed of the plasmid pNAKA62. In another preferred embodiment, the expression vector is composed of the plasmids pNAKA62 and pNAKA52. In another preferred embodiment, the expression vector is composed of the plasmids pNAKA62 and pNAKA53.

In a preferred embodiment, the expression vector is characterised in that it is composed of the plasmid pNAKA143. In another preferred embodiment, the expression vector is composed of the plasmids pNAKA143 and pNAKA53.

Another object described in the present invention relates to a host cell transformed with any of the expression vectors described above. In a preferred embodiment, the cell is preferably a bacterial cell. In another preferred embodiment, the cell is a bacterial cell of the genus *Bacillus,* preferably of the species *B. subtilis.*

In another preferred embodiment, the host cell is selected from any of the following: CECT 7968 and/or CECT 7969 and/or NCIMB 42026.

Another object described in the present invention relates to the use of the CECT 7968 and/or CECT 7969 and/or NCIMB 42026 cells described above for the production of biofuel, preferably biodiesel fuel.

Another object of the present invention relates to a method for producing biofuel which comprises culturing the bacterial strains described in the present invention in the presence of a carbon source, under adequate conditions.

In a preferred embodiment, the method described in the present invention is characterised in that the carbon source is glycerin, preferably glycerin as a by-product.

In another preferred embodiment, the method of the invention is characterised in that it further comprises the isolation of the biofuel produced.

In another preferred embodiment, the method of the invention is characterised in that the isolation of the biofuel is performed by any process available in the state of the art which is usually applied in the sector; preferably, it is performed by means of organic solvents, such as, for example: hexane, ethyl acetate, etc.

In another preferred embodiment, the method of the invention is characterised in that the biofuel obtained is composed of fully saturated branched fatty acid ethyl esters.

In another preferred embodiment, the method of the invention is characterised in that the fatty acid ethyl esters are preferably: iso-C15 (13-methyltetradecanoic acid), anteiso-C15:0 (12-methyltetradecanoic acid), n-C15:0 (n-pentadecanoic acid); iso-C16:0 (14-methylpentadecanoic acid), n-C16:0 (n-hexadecanoic acid), iso-C17:0 (15-methylhexadecanoic acid), anteiso-C17:0 (14-methylhexadecanoic acid), n-C17:0 (n-heptadecanoic acid), iso-C18:0 (16-methylheptadecanoic acid) and n-C18:0 (n-octadecanoic acid).

In another preferred embodiment, the method of the invention is characterised in that the biofuel is biodiesel fuel.

In another preferred embodiment, the method of the invention is characterised in that it further comprises the addition of at least one additive.

Another object of the present invention relates to a biofuel composition that comprises fully saturated branched fatty acid ethyl esters.

In a preferred embodiment, the composition of the invention is characterised in that the fatty acid ethyl esters are formed by fully saturated carbonated chains of the following types: iso-C15 (13-methyltetradecanoic acid), anteiso-C15:0 (12-methyltetradecanoic acid), n-C15:0 (n-pentadecanoic acid); iso-C16:0 (14-methylpentadecanoic acid), n-C16:0 (n-hexadecanoic acid), iso-C17:0 (15-methylhexadecanoic acid), anteiso-C17:0 (14-methylhexadecanoic acid), n-C17:0 (n-heptadecanoic acid), iso-C18:0 (16-methylheptadecanoic acid) and n-C18:0 (n-octadecanoic acid).

In another preferred embodiment, the composition of the invention is characterised in that it is a biodiesel composition and may further comprise at least one adequate additive.

The examples presented below are intended to illustrate the invention without limiting the scope thereof.

### Example 1. Characterisation of the growth of B. subtilis in media containing glycerol recovered from different industrial processes as the carbon source.

In order to corroborate that *B. subtilis* bacteria grow in the presence of glycerol or glycerin as the carbon source, the glycerol content in two samples from industrial processes was quantified by means of a commercial kit. One of the samples came from the processing of soya oil (SO) and the other came from frying oil (FO) discards. In both cases, the glycerol content (purity) was approximately 50% (w/v), whereas commercial glycerin has a percentage purity of 87%. The appearance of SO is clearer than the appearance of FO. Both significantly increase the turbidity of aqueous media, which suggests a significant lipid residue of fatty acids, phospholipids or other organic products. The pH of both oils is approximately 9.

Therefore, the usefulness of both samples as a carbon and energy source was analysed in cultures of wild *B. subtilis* JH642 bacteria in Spizizen's minimal culture medium (MSM) composed of 2.0 g/l (NH₄)₂ SO₄; 14.0 g/l KH₂PO₄; 6.0 g/l K₂HPO₄; 1.0 g/l Na Citrate; 0.2 g/l MgSO₄ 7 H₂O, supplemented with 0.01 % tryptophan, 0.01 % phenylalanine and 0.01 % threonine (final concentrations). Commercial glycerin was used as a control. The growth of the wild *B**. *subtilis* JH642 bacterial cultures could not be directly analysed by measuring the absorbance, due to the turbidity generated by the impurities in the commercial samples. For this reason, aliquots were obtained from the culture medium and centrifuged prior to measuring the absorbance, and they were re-suspended in fresh medium, adding the same volume centrifuged. With time, the bacteria grown in SO reached growth values similar to those obtained for the wild *B. subtilis* JH642 bacteria grown in glycerol, although the latent phase (period of time during which the bacterial inoculum adapts to the conditions of the medium whereon it has been seeded and metabolic adjustment takes place) was longer and the growth rate was lower. On the contrary, the growth of wild *B. subtilis* JH642 bacteria in FO was lower and at a very low rate; for this reason, it was a less adequate source for the growth of wild *B. subtilis* bacteria. Therefore, the present example shows that wild bacterial strains of the species *B. subtilis* are capable of growing in cultures with raw glycerin as the only carbon source.

### Example 2. Obtainment of strains CECT 7968, CECT 7969 and NCIMB 42026 of the species B. subtilis capable of producing biodiesel from glycerin as the carbon source.

A disadvantage in the expression of heterologous genes in bacteria of the species *B*. *subtilis* is the different use of codons of said Gram-positive bacterial species with respect to the use of said codons by other Gram-negative bacterial species. This is due to the different availability of tRNA for each codon in different organisms. In order to overcome said disadvantage and maximise the translation of the *adhB* (SEQ ID NO: 4), *pdc* (SEQ ID NO: 2), *'tesA* (SEQ ID NO: 6 or SEQ ID NO: 17) and *atf1* (SEQ ID NO: 8) heterologous synthetic mutated genes, all originating from Gram-negative bacteria, the original sequences of said genes were mutated in order to promote a greater, more stable expression in *B. subtilis.*

To this end, the codon usage frequency was determined in a number of genes encoding ribosomal proteins (*rplA, rplJ,* with GenBank accession no.: D50303.1; *rplC, rplD, rplB, rpsC,* with GenBank accession no.: U43929.1; *rplF, rpsE,* with GenBank accession no.: L47971.1; *rpsD,* with GenBank accession no.: S45404.1; and *rpsG,* with GenBank accession no.: D64127.1), which are highly expressed in *B. subtilis.* Once the most frequently used codons in the expression of said genes in said bacterial species were known, the codons with a usage frecuency lower than 20% were determined in the sequences of the original genes of interest: *adhB* (SEQ ID NO: 3), *pdc* (SEQ ID NO: 1), *'tesA* (SEQ ID NO: 5) and *atf1* (SEQ ID NO: 7). Those codons that presented a very low expression frequency were replaced with the codon with the greatest usage found in the analysis of the codon usage frequency in the ribosomal genes of *B. subtilis,* always taking into consideration that the new codon introduced must encode the same amino acid as the codon that it replaces, i.e. the substitution of one codon by another must be conservative and not alter the amino acid sequence of the final protein at any time.

In this regard, as discussed above, the heterologous synthetic mutated sequences were obtained for each gene of interest, said sequences being SEQ ID NO: 2 or variants thereof for the *pdc* gene; SEQ ID NO: 4 or variants thereof for the *adhB* gene, SEQ ID NO: 8 or variants thereof for the *atf1* gene, and SEQ ID NOs: 6 and 17 or variants thereof for the *'tesA* gene. For purposes of the present invention, in the present example five optimised sequences are described for their expression in *B. subtilis,* using the method described herein, but any other sequence/s of said genes, obtained by the optimisation method described herein for stable, increased expression in *B. subtilis* may be used, provided that said optimisation of the original gene sequence encodes the same protein, i.e. said optimisation must be conservative and not alter the amino acid sequence of the final protein at any time.

Once the new optimised sequences of the four heterologous genes (SEQ ID NOs: 2, 4, 6, 8 and 17 or variants thereof) were obtained, they were synthesised *in vitro* by the company Blue Heron Biotechnology (Bothell, USA), which supplied the heterologous synthetic mutated genes, each cloned at the SmaI site of the Bluescript SK vector, from which the multiple cloning site was eliminated. For said cloning, extra sequences that encode restriction sites for specific enzymes and the ribosome-binding sequence (rbs) were added to the ends of each of sequences SEQ ID NOs: 2, 4, 6, 8 and 17, or variants thereof. In the present invention, the sequences of the restriction sites for the HindIII, BgIII, BamHI, Xbal and SalI enzymes, amongst others, were used, and any other restriction enzyme known in the state of the art for the same purpose may be used. The optimised sequences of the four heterologous synthetic genes which, moreover, comprise the sequences with the restriction sites for the enzymes and the rbs sequence at the ends thereof are: SEQ ID NO: 18 or variants thereof, for the *pdc* gene, SEQ ID NO: 19 or variants thereof for the *adhB* gene, SEQ. ID NOs. 20-21 or variants thereof for the *'tesA* gene, and SEQ ID NO: 22 or variants thereof for the *atf1* gene. The vectors obtained were subsequently used to transform competent *E. coli DH5a* bacteria in order to preserve and amplify the plasmids.

Subsequently, the heterologous synthetic mutated genes were sub-cloned in the expression vector pDR67 (Ireton K, 1993), to obtain, on the one hand, the plasmid pNAKA62 (pDR67 (HindIII/BglII) + *pdc-adh-atf1-'tesA),* which contains the heterologous synthetic mutated sequences of the four genes of interest, *pdc-adh-atf1-'tesA* (SEQ ID NO: 2-SEQ ID NO: 4-SEQ ID NO: 8-SEQ ID NO: 6, respectively), and, on the other hand, the plasmid pNAKA143 (pDR67 (HindIII/BglII) + *pdc-adh-atf1-'tesA),* which contains the heterologous synthetic mutated sequences of the four genes of interest, *pdc-adh-atf1-'tesA* (SEQ ID NO: 2-SEQ ID NO: 4-SEQ ID NO: 8-SEQ ID NO: 17, respectively). In both plasmids, pNAKA62 and pNAKA143, the four genes are under the control of the Pspac promoter, which is inducible in the presence of isopropyl-β-D-thiogalactoside (IPTG) in the culture medium, but any other plasmid known in the state of the art for the same purpose may be used, under the control of an inducible or constitutive promoter, depending on the process requirements. Moreover, said plasmids, pNAKA62 and pNAKA143, contain the initial and final regions of the *amyE* gene, flanking the operon, and a chloramphenicol antibiotic resistance cassette.

Using the plasmid pNAKA62, wild strains of *B. subtilis* JH642 were transformed, to obtain the strain called LN72 (JH642/pNAKA62). In the same manner, using the plasmid pNAKA143, wild strains of *B. subtilis* JH642 were transformed, to obtain the strain called LN145 (JH642/pNAKA143). The insertion of the plasmid in the *amyE* locus was confirmed by the loss of amylase activity in the strain obtained. Therefore, said strains LN72 and LN143 are capable of expressing the *pdc* (SEQ ID NO: 2), *adh* (SEQ ID NO: 4), *atf1* (SEQ ID NO: 8) and *'tesA* (SEQ ID NO: 6) heterologous synthetic genes, in the case of strain LN72, and the *pdc* (SEQ ID NO: 2), *adh* (SEQ ID NO: 4), *atf1* (SEQ ID NO: 8) and *'tesA* (SEQ ID NO: 17) heterologous synthetic genes, in the case of strain LN145, thus being capable of increasing the synthesis of fatty acids and ethanol, in the presence of glycerin as the carbon source, and producing biodiesel.

In order to increase the yield of biodiesel synthesis by means of the strains described in the present invention, it is advisable to overexpress at least one of the genes that form the acyl-Co ligase complex (E.C.6.2.1.3) and the ACC complex (E.C.6.4.1.2). For the overexpression of at least one of the genes of the acyl-CoA ligase enzymatic complex (E.C.6.2.1.3), the *lcfA* and *yhfL* genes were chosen. Briefly, said genes were amplified by PCR, using chromosomal DNA from the wild strain of *B. subtilis* JH642 as the template and those defined as SEQ ID NOs: 9-13 as the primers. To this end, the plasmid pNAKA60 was constructed by sub-cloning the *lacI* gene, which encodes the Lac repressor, and the Pspac promoter^{hy} in the vector pDG1731 (Guérout-Fleury AM., 1996). Said plasmid pNAKA60 contains the initial and final regions of the *thrC* gene, flanking *lacI-Pspac^{hy}*, and a spectinomycin antibiotic resistance cassette. Subsequently, the *lcfA* and *yhfL* genes, which encode acyl-CoA-ligases, were sub-cloned in said plasmid, to generate the plasmids pNAKA52 and pNAKA53, respectively. With said constructs, pNAKA52 and pNAKA53, the LN72 strains were transformed, to obtain strains CECT 7968 and CECT 7969, respectively. In the same way, using construct pNAKA53, the LN145 strains were transformed, to obtain strain NCIMB 42026. The integration of said genes in the *trhC* locus was confirmed by the generation of auxotrophy for the amino acid threonine; therefore, the strains became auxotrophic for said amino acid. Moreover, the four synthetic mutated genes are expressed in the *amyE* locus, for which reason said strains are no longer able to degrade starch. Therefore, strains CECT 7968, CECT 7969 and NCIMB 42026 contain the *amyE* and *thrC* loci with all the necessary genes for the biosynthesis of biodiesel under the control of the IPTG inducible promoter Pspac, although, as discussed throughout the present invention, any promoter, whether inducible or constitutive, known in the state of the art may be selected.

In the same manner as mentioned above, in order to overexpress the four subunits of the acetyl-CoA carboxylase enzymatic complex (AccDABC) in the strains described in the present invention, and thus achieve, if so desired, a higher yield of biodiesel synthesis, the same protocol described for the overexpression of the enzymes of the Acyl-CoA ligase complex was followed. Briefly, the *accDA* and *accBC* genes were amplified by PCR, using chromosomal DNA from the wild strain of *B. subtilis* JH642 as the template and those defined as SEQ ID NOs: 13-16 as the primers. Successive clonings were performed in order to finally obtain the plasmid pGES468, which contains the operon *accDABC* under the control of the IPTG inducible promoter P*spac,* although, as mentioned above, any promoter, whether inducible or constitutive, known in the state of the art may be selected. By means of said plasmid, if so desired, *B. subtilis* strains CECT 7968, CECT 7969 and NCIMB 42026 are transformed, in order to increase the expression of the acetyl-CoA carboxylase enzymatic complex and increase the yield of biodiesel synthesis by means of the strains described in the present invention.

### Example 3. Production of biodiesel by means of B. subtilis strains CECT 7968, 7969 and NCIMB 42026.

*B. subtilis* strains CECT 7968 and CECT 7969 described in the present invention were cultured in 50 ml of MSM minimal culture medium composed of 2.0 g/l (NH₄)₂ SO₄; 14.0 g/l KH₂PO₄; 6.0 g/l K₂HPO₄; 1.0 g/l Na Citrate; 0.2 g/l MgSO₄ 7H₂O, supplemented with 0.5% glucose; 0.01% tryptophan; 0.01% phenylalanine; and 0.01% threonine (final concentrations), and 500 µM IPTGM at 37°C until the stationary phase (OD₆₀₀ₙₘ = 1.5). Subsequently, a 10-ml volume of hexane was added to each culture and the lipids synthesised by said bacterial strains were extracted at room temperature for 20 min. The extraction may be performed by means of organic extraction processes in the presence of any compound of said nature as the solvent, such as, for example, hexane or ethyl acetate. After centrifuging, the organic phase was evaporated by means of a stream of N₂ at room temperature, and, subsequently, the composition of the biofuel obtained was analysed by means of gas chromatography coupled to mass spectrometry (GC-MS).

Said analysis of the composition of the biofuel obtained showed the presence of fatty acid ethyl esters or biodiesel, with a fully saturated carbonated chain, preferably formed by: iso-C15 (13-methyltetradecanoic acid), anteiso-C15:0 (12-methyltetradecanoic acid), n-C15:0 (n-pentadecanoic acid); iso-C16:0 (14-methylpentadecanoic acid), n-C16:0 (n-hexadecanoic acid), iso-C17:0 (15-methylhexadecanoic acid), anteiso-C17:0 (14-methylhexadecanoic acid), n-C17:0 (n-heptadecanoic acid), iso-C18:0 (16-methylheptadecanoic acid) and n-C18:0 (n-octadecanoic acid). These species made up approximately 10% of the lipid sample, the rest being primarily free fatty acids. The ethyl esters were identified using standards and confirmed by their mass spectra. As may be observed, said biodiesel produced is mostly composed of saturated branched fatty acids, unlike other biofuels produced, for example, by strains of *E. coli,* which are primarily composed of linear fatty acids with an even number of carbon atoms, on the average, 50%, with one unsaturation. Both extracts obtained from the cultures of strains CECT 7968 and CECT 7969 described in the present invention presented the same composition.

In order to obtain biodiesel using strain NCIMB 42026, the same strategy described above for strains CECT 7968 and CECT 7969 was followed, the only difference being the optical density of IPTG, which, instead of being OD₆₀₀ₙₘ = 1.5, as in the case of strains CECT 7968 and 7969, was OD₆₀₀ₙₘ = 0.6 for strain NCIMB 42026. The analysis of the biodiesel obtained by means of strain NCIMB 42026 showed that it presented a composition of ethyl esters similar to that obtained by using strains CECT 7968 and 7969, which was described above.

### BIBLIOGRAPHY

1. Atsumi S, Liao JC. Metabolic engineering for advanced biofuels production from Escherichia coli. Curr Opin Biotechnol. 2008 Oct; 19(5): 414-9.
2. Karmakar A, Karmakar S, Mukherjee S. Properties of various plants and animals feedstocks for biodiesel production. Bioresource Technology. 2010 Oct; 101 (2010) 7201-10.
3. Kalscheuer R, Stólting T, Steinbüchel A. Microdiesel: Escherichia coli engineered for fuel production. Microbiology. 2006 Sep; 152: 2529-36.
4. Shams Yazdani S, González R. Engineering Escherichia coli for the efficient conversion of glycerol to ethanol and co-products. Metab Eng. 2008 Nov; 10(6): 340-51.
5. da Silva GP, Mack M, Contiero. Glycerol: a promising and abundant carbon source for industrial microbiology. J. Biotechnol Adv. 2009; 27(1): 30-9.
6. Choi WJ. Glycerol-based biorefinery for fuels and chemicals. Recent Pat Biotechnol. 2008; 2(3): 173-80.
7. Schujman GE, Altabe S, de Mendoza D. A malonyl-CoA-dependent switch in the bacterial response to a dysfunction of lipid metabolism. Molecular Microbiology. 2008; 68 (4): 987-996.
8. Davis, M.S., Solbiati, J., Cronan, J.E., Jr. Overproduction of acetyl-CoA carboxylase activity increases the rate of fatty acid biosynthesis in Escherichia coli. J. Biol. Chem. 2000; 275: 28593-28598.
9. Kalscheuer, R., T. Stolting, A. Steinbuchel. Microdiesel: Escherichia coli engineered for fuel production. Microbiology. 2006; 152: 2529-36.
10. Ireton K, Rudner DZ, Siranosian KJ and Grossman AD. Integration of multiple developmental signals in Bacillus subtilis through the SpoOA transcription factor. Genes & Dev. 1993 7: 283-294.
11. Guérout-Fleury AM, Frandsen N, Stragier P. Plasmids for ectopic integration in Bacillus subtilis. Gene 180 (1996) 57-61.
12. Oh BR, Seo JW, Heo SY, Hong WK, Luo LH, Joe MH, Park DH, Kim CH. Efficient production of ethanol from crude glycerol by a Klebsiella pneumoniae mutant strain. Bioresour Technol. 2011 Feb; 102(4): 3918-22.
13. Westers et al. Biochimica et Biophysica Acta 1694 (2004): 299-310.

## Claims

1. Gene construct that comprises at least the genes **characterised by** SEQ ID NO: 2 or SEQ ID NO: 18, SEQ ID NO: 4 or SEQ ID NO: 19, SEQ ID NO: 6 or SEQ ID NO: 17 or SEQ ID NO: 20 or SEQ ID NO: 21 and SEQ ID NO: 8 or SEQ ID NO: 22, or variants thereof, jointly with at least one promoter.

2. Gene construct according to claim 1, **characterised in that** it comprises at least the genes **characterised by** SEQ ID NO: 2, SEQ ID NO: 4, SEQ ID NO: 6 or SEQ ID NO: 17 and SEQ ID NO: 8, or variants thereof, jointly with at least one promoter.

3. Gene construct according to claim 1, **characterised in that** it comprises at least the genes **characterised by** SEQ ID NO: 18, SEQ ID NO: 19, SEQ ID NO: 20 or SEQ ID NO: 21 and SEQ ID NO: 22, or variants thereof, jointly with at least one promoter.

4. Gene construct according to any of claims 1 to 3, **characterised in that** it further comprises at least one of the nucleotide sequences, or variants thereof, that encode the genes selected from: *lcfA, yhfL, yhfT, accD, accA, accB* or *accC* and/or any combination thereof.

5. Gene construct according to claim 4, **characterised in that** the nucleotide sequences or variants thereof, which encode the *lcfA, yhfL, yhfT, accD, accA, accB* or *accC* genes belong to the bacterial species *B. subtilis.*

6. Gene construct according to any of claims 1 to 5, **characterised in that** the promoter is inducible or constitutive.

7. Gene construct according to claim 6, **characterised in that** the inducible promoter is selected from the Pspac promoter or the Pspachy promoter.

8. Gene construct according to claim 6, **characterised in that** the constitutive promoter is the Pcap promoter.

9. Gene construct according to any of the preceding claims, **characterised in that** it is selected from any of the following: pNAKA62, pNAKA143, pNAKA52, pNAKA53 and/or combinations thereof.

10. Expression vector that comprises sequences SEQ ID NO: 2 or SEQ ID NO: 18, SEQ ID NO: 4 or SEQ ID NO: 19, SEQ ID NO: 6 or SEQ ID NO: 17 or SEQ ID NO: 20 or SEQ ID NO: 21 and SEQ ID NO: 8 or SEQ ID NO: 22 or variants thereof.

11. Expression vector according to claim 10, **characterised in that** it comprises sequences SEQ ID NO: 2, SEQ ID NO: 4, SEQ ID NO: 6 or SEQ ID NO: 17 and SEQ ID NO: 8 or variants thereof.

12. Expression vector according to claim 10, **characterised in that** it comprises sequences SEQ ID NO: 18, SEQ ID NO: 19, SEQ ID NO: 20 or SEQ ID NO: 21 and SEQ ID NO: 22 or variants thereof.

13. Expression vector according to any of claims 10 to 12, **characterised in that** it further comprises at least one of the nucleotide sequences, or variants thereof, which encode the genes selected from: *lcfA, yhfL, yhfT, accD, accA, accB* or *accC* and/or any combination thereof.

14. Expression vector according to claim 13, **characterised in that** the nucleotide sequences, or variants thereof, which encode the *lcfA, yhfL, yhfT, accD, accA, accB* or *accC* genes belong to the bacterial species *B. subtilis.*

15. Expression vector according to any of claims 10 to 14, **characterised in that** it comprises at least one of the gene constructs according to claims 1 to 9.

16. Expression vector according to claim 15, which is composed of the plasmid pNAKA62.

17. Expression vector according to claim 15, which is composed of the plasmids pNAKA62 and pNAKA52.

18. Expression vector according to claim 15, which is composed of the plasmids pNAKA62 and pNAKA53.

19. Expression vector according to claim 15, which is composed of the plasmid pNAKA143.

20. Expression vector according to claim 15, which is composed of the plasmids pNAKA143 and pNAKA53.

21. Cell transformed with any of the expression vectors of claims 10 to 20.

22. Cell according to claim 21, **characterised in that** it is a bacterial cell.

23. Cell according to claim 22, **characterised in that** it is a bacterial cell of the genus *Bacillus.*

24. Cell according to claim 23, **characterised in that** it is of the species *B. subtilis.*

25. Cell according to any of claims 21 to 24, **characterised in that** it is selected from any of the following: CECT 7968, CECT 7969 or NCIMB 42026.

26. Use of the cells of claims 21 to 25 for the production of biofuel.

27. Use according to claim 26, **characterised in that** the biofuel is biodiesel fuel.

28. Method for producing biofuel, which comprises culturing the cells of claims 21 to 25 in the presence of a carbon source, under adequate conditions.

29. Method according to claim 28, where the carbon source is glycerin.

30. Method according to claim 29, **characterised in that** the glycerin used as the carbon source is a by-product.

31. Method according to claims 28 to 30, which further comprises the isolation of the biofuel produced.

32. Method according to claim 31, where the isolation is performed by organic extraction processes.

33. Method according to claims 28 to 32, **characterised in that** the biofuel obtained is composed of saturated branched fatty acids.

34. Method according to claim 33, **characterised in that** the fatty acids are preferably: iso-C15:0, n-c15:0, anteiso-C15:0, iso-C16:0, n-C16:0, iso-C17:0, n-c17:0, anteiso-C17:0 and n-C18:0.

35. Method according to claims 28 to 34, **characterised in that** the biofuel is biodiesel fuel.

36. Method according to any of claims 28 to 35, **characterised in that** it further comprises the addition of, at least, one additive.

37. Biofuel composition that comprises saturated branched fatty acid ethyl esters.

38. Composition according to claim 37, **characterised in that** the fatty acid ethyl esters are: iso-C15:0, n-c15:0, anteiso-C15:0, iso-C16:0, n-C16:0, iso-C17:0, n-c17:0, anteiso-C17:0 and n-C18:0.

39. Composition according to claims 37 to 38, **characterised in that** the biofuel is biodiesel fuel.

40. Composition according to claims 37 to 39, **characterised in that** it further comprises, at least, one additive.
